Europäisches Patentamt

(19) European Patent Office

Office européen des brevets

(11) **EP 0 761 204 B1**

(12) **FASCICULE DE BREVET EUROPEEN**

(45) Date de publication et mention
de la délivrance du brevet:
**15.04.1998 Bulletin 1998/16**

(51) Int. Cl.$^6$: **A61K 7/48**, A61K 35/74,
A61K 35/78, A61K 7/06

(21) Numéro de dépôt: 96401781.8

(22) Date de dépôt: **13.08.1996**

(54) **Utilisation d'un extrait d'une bactérie filamenteuse non photosynthétique et composition le contenant**

Verwendung von Extrakten aus filamentösen, nicht photosynthetischen Bakterien sowie ihre Zusammensetzungen

Use of extracts of filamentous, non photosynthetic bacteria and the composition containing them

(84) Etats contractants désignés:
**DE ES FR GB IT SE**

(30) Priorité: **07.09.1995 FR 9510485**
**27.03.1996 FR 9603816**
**27.03.1996 FR 9603818**

(43) Date de publication de la demande:
**12.03.1997 Bulletin 1997/11**

(73) Titulaire: **L'OREAL**
**75008 Paris (FR)**

(72) Inventeurs:
• **Breton, Lionel**
**78000 Versailles (FR)**
• **Aubert, Lucien**
**Chemin du Bautugan, 06320 Cap d'Ail (FR)**
• **Leclaire, Jacques**
**91300 Massy (FR)**
• **Martin, Richard**
**37210 Rochecorbon (FR)**
• **De Lacharriere, Olivier**
**75015 Paris (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 681 831        EP-A- 0 692 247**
**FR-A- 2 283 223        FR-A- 2 283 223**
**FR-A- 2 693 654        GB-A- 2 034 687**

• **PATENT ABSTRACTS OF JAPAN vol. 012, no. 181 (C-499), 27 Mai 1988 & JP-A-62 289509 (SHISEIDO CO LTD), 16 Décembre 1987,**
• **PATENT ABSTRACTS OF JAPAN vol. 011, no. 259 (C-441), 21 Août 1987 & JP-A-62 061924 (SHISEIDO CO LTD), 18 Mars 1987,**
• **PATENT ABSTRACTS OF JAPAN vol. 95, no. 001 & JP-A-07 025762 (SANSHO SEIYAKU CO LTD), 27 Janvier 1995,**

**Description**

La présente invention concerne l'utilisation d'au moins un extrait d'au moins une bactérie filamenteuse non photosynthétique en tant qu'antagoniste de substance P, dans une composition cosmétique ou pour la préparation d'une composition pharmaceutique. L'invention concerne également l'utilisation d'au moins un extrait d'au moins une bactérie filamenteuse non photosynthétique dans une composition cosmétique ou pour la préparation d'une composition pharmaceutique destinée à traiter les désordres associées à un excès de synthèse et/ou de libération de substance P. L'invention concerne en outre diverses compositions contenant un extrait d'au moins une bactérie filamenteuse non photosynthétique et un procédé de traitement cosmétique.

Il existe chez les mammifères des polypeptides appartenant à la famille des tachykinines qui induisent sur les fibres musculaires lisses des contractions rapides. Parmi les composés de cette famille on peut citer la neurokinine $\beta$, neurokinine $\alpha$ et la substance P.

La substance P est un élément chimique polypeptidique (undécapeptide), élaboré et libéré par une terminaison nerveuse. La localisation de la substance P est spécifique des neurones, tant dans le système nerveux central que dans les organes à la périphérie. Ainsi, de très nombreux organes ou tissus reçoivent des afférences de neurones à substance P, il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment des systèmes nerveux central et périphérique.

De par la distribution ubiquitaire de la substance P, de très nombreux désordres sont associés à un excès de synthèse et / ou de libération de substance P.

La substance P intervient notamment dans la transmission de la douleur et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie), dans des maladies respiratoires (telles que par exemple les broncho-pneumonies) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn), dans des désordres cutanés (tels que par exemple le psoriasis, les maladies prurigineuses, l'herpès, les photodermatoses, les dermatites atopiques, les dermatites de contact, les lichens, les prurigos, les prurits, les piqûres d'insectes), dans des fibroses et autres troubles de la maturation des collagènes (tels que par exemple la sclérodermie), dans des troubles cardio-vasculaires, dans des troubles vasospastiques (tels que par exemple les migraines, la maladie de Reynaud), dans des désordres immunologiques, dans des troubles du tractus urinaire (tels que par exemple l'incontinence, la cystite), dans des maladies rhumatismales dans certaines maladies dermatologiques (telles que l'eczéma) et dans les affections ophtalmologiques (telles que par exemple les conjonctivites, les uvéites, les prurits oculaires, les douleurs oculaires et les irritations).

L'utilisation d'antagoniste de substance P est l'une des alternatives thérapeutiques efficaces dans toutes les affections citées ci-dessus.

Par antagoniste de substance P, on entend tout composé susceptible d'inhiber partiellement, voire totalement, l'effet biologique de la substance P. Particulièrement, pour qu'une substance soit reconnue comme un antagoniste de substance P elle doit induire une réponse pharmacologique cohérente (incluant ou non sa fixation au récepteur de la substance P) notamment dans l'un des tests suivants :

- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

A ce jour, des antagonistes de substance P sont utilisés pour traiter les désordres indiqués ci-dessus. A cet effet, on peut se référer aux documents US-A-4472305, US-A-4839465, EP-A-101929, EP-A-333174, EP-A-336230, EP-A-394989, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116, EP-A-522808 et WO-A-93/01165.

Cependant aucun de ces documents n'envisage, ni ne suggère qu'un extrait d'au moins une bactérie filamenteuse non photosynthétique puisse avoir une activité antagoniste de substance P telle que définie ci-dessus et donc puisse être notamment utilisé pour traiter les désordres indiqués ci-dessus.

En effet les extraits de bactéries filamenteuses non photosynthétiques sont connus pour leur utilisation comme agents cosmétiques contre le vieillissement cutané (EP-A-681831), pour leur utilisation comme médicament notamment immunomodulateur (FR 2 693 654) ou encore pour leur utilisation dans des compositions à usage topique contenant un antioxydant (EP-A-692247)

La demanderesse a découvert qu'un extrait de bactéries filamenteuses non photosynthétiques répond aux caractéristiques définies comme caractérisant un antagoniste de substance P et peut donc être utilisé comme antagoniste de substance P.

Ainsi, l'invention concerne l'utilisation d'au moins un extrait d'au moins une bactérie filamenteuse non photosynthétique comme antagoniste de substance P dans une composition cosmétique ou pour la préparation d'une composition pharmaceutique.

L'invention concerne également l'utilisation d'au moins un extrait d'au moins une bactérie filamenteuse non photosynthétique dans une composition cosmétique ou pour la préparation d'une composition pharmaceutique destinée à traiter les désordres associées à un excès de synthèse et/ou de libération de substance P.

Par extrait de bactéries filamenteuses non photosynthétiques, on entend aussi bien le surnageant de culture desdites bactéries, la biomasse obtenue après culture desdites bactéries ou encore les extraits de la biomasse obtenus par traitement de cette biomasse.

Les extraits de bactéries selon l'invention sont préparés à partir de bactéries filamenteuses non photosynthétiques telles que définies selon la classification du Bergey's Manual of Systematic Bacteriology (vol. 3, sections 22 et 23, 9°édition, 1989), parmi lesquelles on peut citer les bactéries appartenant à l'ordre des Beggiatoales, et plus particulièrement les bactéries appartenant aux genres Beggiatoa, Vitreoscilla, Flexithrix ou Leucothrix.

Les bactéries qui viennent d'être définies et dont plusieurs ont déjà été décrites ont généralement un habitat aquatique et peuvent être trouvées notamment dans des eaux marines ou dans des eaux thermales. Parmi les bactéries utilisables, on peut citer par exemple :

*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)

Préférentiellement, on utilise selon l'invention une souche de *Vitreoscilla filiformis*.

Pour préparer l'extrait selon l'invention, on peut cultiver lesdites bactéries selon les méthodes connues de l'homme du métier, puis les séparer de la biomasse obtenue, par exemple par filtration, centrifugation, coagulation et/ou lyophilisation. On peut notamment préparer les extraits utilisables selon l'invention, selon le procédé décrit par la demanderesse dans la demande de brevet WO-9402158.

Ainsi, après culture, les bactéries sont concentrées par centrifugation. La biomasse obtenue est autoclavée. Cette biomasse peut être lyophilisée pour constituer ce que l'on appelle l'extrait lyophilisé. Toute méthode de lyophilisation connue de l'homme du métier est utilisable pour préparer cet extrait.

La fraction surnageante de cette biomasse peut également être filtrée dans un récipient stérile pour éliminer les particules en suspension. On obtient ainsi l'extrait dénommé par ailleurs dans le texte extrait aqueux.

Quelque soit sa forme utilisée dans l'invention, la quantité d'extrait bactérien selon l'invention contenue dans la composition est bien entendu fonction de l'effet recherché et peut donc varier dans une large mesure.

Pour donner un ordre de grandeur dans une composition cosmétique ledit extrait bactérien est utilisé en une quantité représentant de 0,0001 % à 20 % du poids total de la composition et de préférence en une quantité représentant de 0,001 % à 10% du poids total de la composition.

Pour donner un ordre de grandeur dans une composition pharmaceutique ledit extrait bactérien est utilisé en une quantité représentant de 0,0001 % à 30 % du poids total de la composition et de préférence en une quantité représentant de 0,05 % à 20 % du poids total de la composition.

On a vu préalablement dans le texte des exemples de désordres liés à un excès de synthèse et/ou de libération de substance P.

Ainsi, selon un aspect particulier, l'invention a pour objet l'utilisation d'au moins un extrait d'au moins une bactérie filamenteuse non photosynthétique dans une composition cosmétique ou pour la préparation d'une composition pharmaceutique destinée à traiter les désordres du système nerveux central, les troubles respiratoires, les syndromes allergiques, l'inflammation, la douleur, les désordres gastro-intestinaux, les désordres cutanés, les fibroses, les troubles de la maturation du collagène les troubles cardio-vasculaires, les troubles vasospastiques, les désordres immunologiques ainsi que les troubles du tractus urinaire.

Dans le domaine des désordres cutanés, il est connu que certaines peaux sont plus sensibles que d'autres. On sait qu'il existe au niveau cutané de nombreux phénomènes d'intolérance dont les symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

Ces phénomènes peuvent être la conséquence d'événements multiples dont les plus banaux seront qualifiés d'irritation ou d'inflammation, mais dont certains seront dus à des causes physiologiques, comme les peaux sensibles, voire même pathologiques comme par exemple, l'allergie.

Toutefois les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques. On assimilait également les peaux sensibles à des peaux allergiques.

Des tests ont été mis au point pour cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217.

Du fait de la méconnaissance des caractéristiques des peaux sensibles, il était jusqu'à présent très difficile voire impossible de les traiter. En fait, on les traitait indirectement, par exemple en limitant dans les compositions cosmétiques ou dermatologiques l'emploi de produits à caractère irritant tels que les tensioactifs, les conservateurs ou les parfums ainsi que l'emploi de certains actifs cosmétiques ou dermatologiques.

Après de nombreux tests cliniques, la demanderesse a pu déterminer les symptômes liés aux peaux sensibles.

La demanderesse a trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température où la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certains produits cosmétiques. En général, ces signes sont associés à une peau hyperséborrhéique ou acnéique avec ou-sans dartres, et à un érythème.

Ces phénomènes peuvent être généralisés à l'ensemble du corps, mais la plupart du temps ils peuvent avoir des localisations bien définies telles que par exemple le cuir chevelu, le visage, les plis cutanés, etc....

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparations cosmétiques, à l'eau dure à forte concentration en calcaire et/ou aux variations de température.

L'ensemble de ces phénomènes d'intolérance est toujours lié à un processus inflammatoire classique, et plus particulièrement à une réaction inflammatoire de type neurogène puisqu'elle fait intervenir les fibres nerveuses cutanées.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. Par contre, la résultante finale d'une réaction allergique se traduit également par une réaction inflammatoire aiguë associé généralement à un oedème.

La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

Quelque soit le phénomène envisagé, il existe un point commun à tous ces mécanismes qui se traduit par une réaction inflammatoire dont la facette terminale se mesure par la libération par les cellules mastocytaires de la peau d'au moins un médiateur de l'inflammation tels que l'histamine, la sérotonine, l'héparine, les leukotriènes, les prostaglandines, les cytokines, le monoxyde d'azote ou des espèces oxygénées réactives.

Pour déterminer si une peau est sensible ou non, la demanderesse a également mis au point un test. En effet,

4

après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm$^2$ de peau 0,05 ml d'une crème comprenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par leur libération sont dites "neurogènes".

Personne jusqu'à ce jour n'avait établi un lien entre la substance P et la peau sensible. Les signes cliniques de la peau sensible sont essentiellement subjectifs : picotements, fourmillements, prurits, tiraillements, échauffements, et ils s'associent parfois à des érythèmes. Ces signes sont dus à des facteurs extérieurs aspécifiques. Les symptômes apparaissent essentiellement localisés au visage, au cou et au cuir chevelu, mais peuvent apparaître aussi sur tout le corps.

Ainsi la demanderesse a découvert que l'une des caractéristiques essentielles des peaux sensibles est liée à la libération de substance P et donc que l'utilisation d'antagonistes de substance P peut permettre d'obtenir un effet préventif et/ou curatif des peaux sensibles.

Pour traiter les peaux sensibles, la demanderesse a donc envisagé d'utiliser des antagonistes de substance P. Elle a en effet constaté de manière surprenante que l'incorporation d'un antagoniste de substance P dans une composition destinée à un usage topique permet d'éviter l'irritation et/ou les sensations dysesthésiques et/ou les prurits de la peau. L'invention concerne donc plus particulièrement l'utilisation d'au moins un extrait d'au moins une bactérie filamenteuse non photosynthétique dans une composition cosmétique ou pour la préparation d'une composition pharmaceutique destinées à traiter les peaux sensibles.

La présente invention a encore pour objet l'utilisation d'au moins un extrait d'au moins une bactérie filamenteuse non photosynthétique dans une composition cosmétique ou pour la préparation d'une composition pharmaceutique destinées à prévenir et/ou à lutter contre les irritations cutanées et/ou les dartres et/ou les érythèmes et/ou les sensations d'échauffement et/ou de dysesthésie et/ou les prurits de la peau et/ou les muqueuses.

De façon avantageuse, selon l'invention au moins un extrait d'au moins une bactérie filamenteuse non photosynthétique peut être associé à des produits à effet irritant utilisés couramment dans le domaine cosmétique ou pharmaceutique, produits qui sont parfois des actifs cosmétiques ou pharmaceutiques. La présence d'un antagoniste de substance P sous la forme d'au moins un extrait d'au moins une bactérie filamenteuse non photosynthétique dans une composition cosmétique ou pharmaceutique comprenant un produit ayant un effet irritant permet d'atténuer fortement, voire de supprimer cet effet irritant. Cela permet en outre d'augmenter la quantité d'actif à effet irritant par rapport à la quantité d'actif normalement utilisée, en vue d'une efficacité améliorée.

Ainsi, l'invention concerne également l'utilisation d'au moins un extrait d'au moins une bactérie filamenteuse non photosynthétique dans une composition cosmétique ou pharmaceutique comprenant en outre au moins un produit à effet irritant.

L'invention concerne également une composition cosmétique ou pharmaceutique comprenant dans un milieu cosmétiquement ou pharmaceutiquement acceptable au moins un extrait d'au moins une bactérie filamenteuse non photosynthétique et en outre au moins un produit à effet irritant, .

Comme produits à effet irritant on peut citer par exemple les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxy-acides (l'acide salicylique et ses dérivés), les α-céto-acides, les β-céto-acides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, les kératolytiques, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

L'emploi d'antagoniste de substance P permet notamment de multiplier de 2 à 10 fois la quantité d'actif à effet irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, en diminuant notablement leur caractère irritant.

Dans certains cas l'ensemble du mécanisme est également sous le contrôle des terminaisons nerveuses sensitives qui libèrent des neuropeptides, notamment la substance P et le CGRP.

Le CGRP, peptide dérivé de la calcitonine (connu sous le nom Calcitonine Gene Related Peptide ou CGRP) est un élément chimique polypeptidique élaboré et libéré par une terminaison nerveuse.

La localisation du CGRP est spécifique des fibres nerveuses sensitives (fibres C). Ainsi, de très nombreux organes ou tissus reçoivent des afférences de neurones à CGRP, il s'agit notamment des glandes salivaires, de l'estomac, du pancréas, de l'intestin, du système cardio-vasculaire, de la glande thyroïde et de la peau.

Un autre but de la présente invention est d'obtenir un effet bénéfique le plus étendu possible dans le traitement de toutes ces affections cutanées et donc de proposer une composition qui agit sur plusieurs composantes de ces affections.

Ainsi, selon un autre aspect, la présente invention a pour objet l'utilisation d'au moins un extrait d'au moins une bactérie filamenteuse non photosynthétique tel que décrit précédement dans une composition cosmétique ou pharmaceutique comprenant en outre au moins un extrait de cellules d'au moins un végétal de la famille des Iridacées.

Selon un autre aspect, la présente invention a pour objet une composition cosmétique ou pharmaceutique, caractérisée en ce qu'elle comprend, dans un milieu cosmétiquement ou pharmaceutiquement acceptable, un extrait d'au moins une bactérie filamenteuse non photosynthétique tel que décrit précédement et un extrait de matériel végétal d'au moins une Iridacée.

L'extrait d'au moins une Iridacée peut être tout extrait préparé à partir de matériel végétal issu de la famille des Iridacées.

La composition peut contenir un extrait d'au moins une Iridacée obtenu à partir de matériel végétal issu de plante entière cultivée *in vivo* ou issu de culture *in vitro.*

Ainsi, par exemple selon l'invention l'extrait peut être un extrait d'organe voire de cellules d'organe d'au moins une Iridacée (racines, tige, feuille) ou encore un extrait de cellules indifférenciées d'au moins une Iridacée.

De préférence on utilise un extrait obtenu à partir de cellules indifférenciées obtenues par culture *in vivo* ou *in vitro.*

La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Par culture *in vitro,* on entend l'ensemble des techniques connues de l'homme du métier qui permet de manière artificielle l'obtention d'un végétal ou d'une partie d'un végétal.

On peut ainsi utiliser par exemple selon l'invention un extrait de racines d'au moins une Iridacée cultivées *in vitro* ou encore un extrait de cellules indifférenciées d'au moins une Iridacée.

De préférence, on utilise un extrait obtenu à partir de matériel végétal cultivé *in vitro* et encore plus préférentiellement un extrait obtenu à partir de cellules indifférenciées cultivées *in vitro.*

Par cellules végétales indifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.

Selon la méthode de culture choisie, et en particulier selon le milieu de culture choisi, il est possible d'obtenir, à partir d'un même explant, des cellules végétales indifférenciées présentant des caractères différents.

La famille des Iridacées (ou Iridées) compte environ 750 espèces.

Les plantes de la famille des Iridacées sont surtout utilisées pour leur propriétés aromatiques et ornementales.

Parmi les genres d'Iridacées utilisables selon l'invention, on peut citer à titre d'exemple les genres Romulea, Crocus, Iris, Gladiolus, Sisyrinchium ou encore Hermodactylus.

Comme matériel végétal utilisable on peut citer celui provenant *d'Iris germanica*, *d'Iris florentina*, *d'Iris pallida,* de *Crocus versicolor,* de *Romulea bulbucodium* ou encore de *Gladiolus communis.*

Plus particulièrement, selon l'invention, on utilise du matériel végétal issu du genre Iris et préférentiellement du matériel végétal *d'Iris pallida.*

Toute méthode d'extraction connue de l'homme du métier peut être utilisée pour préparer l'extrait contenu dans la composition selon l'invention. On peut, en particulier, citer les extraits alcooliques, notamment éthanoliques ou encore hydroalcooliques.

On peut également utiliser un extrait préparé par la méthode décrite dans la demande de brevet français n° 95-02379 déposée par la demanderesse.

Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait. D'autre part, la première étape peut avantageusement être remplacée par une opération de congélation simple des tissus végétaux (par exemple à -20°C), suivie d'une extraction aqueuse reprenant les deuxième et troisième étapes ci-dessus décrites.

Un exemple de préparation d'extrait d'Iridacées utilisable selon l'invention est donné par ailleurs dans les exemples.

La quantité d'extrait contenue dans la composition de l'invention est bien entendu fonction de l'effet recherché et peut donc varier dans une large mesure.

5Pour donner un ordre de grandeur, si la composition est une composition cosmétique, elle peut contenir un extrait d'au moins une Iridacée en une quantité représentant de 0,001% à 20% du poids total de la composition et préférentiellement en une quantité représentant de 0,01% à 10% du poids total de la composition.

Pour donner un ordre de grandeur, si la composition est une composition pharmaceutique, elle peut contenir un extrait d'au moins une Iridacée en une quantité représentant de 0,01% à 30% du poids total de la composition et préférentiellement en une quantité représentant de 0,5% à 20% du poids total de la composition.

Selon encore un autre aspect la présente invention a pour objet l'utilisation d'un extrait d'au moins une bactérie filamenteuse non photosynthétique tel que décrit précédement dans une composition cosmétique ou pharmaceutique comprenant en outre au moins un composé diminuant la synthèse, la libération et/ou l'activité d'au moins un médiateur de l'inflammation à l'exception des agents anti-inflammatoires stéroïdiens et non-stéroïdiens.

L'invention concerne également une composition cosmétique ou pharmaceutique, caractérisée en ce qu'elle comprend, dans un milieu cosmétiquement ou pharmaceutiquement acceptable, un extrait d'au moins une bactérie filamenteuse non photosynthétique tel que décrit précédement et un composé diminuant la synthèse, la libération et/ou l'activité d'au moins un médiateur de l'inflammation à l'exception des agents anti-inflammatoires stéroïdiens et non-stéroïdiens.

Parmi les agents anti-inflammatoires stéroïdiens on peut citer à titre d'exemple l'hydrocortisone, le valérate de bétaméthasone ou le propionate de clobétasol.

Par agents anti-inflammatoires non-stéroïdiens, on entend ici les agents anti-inflammatoires tels que décrits par Schorderet et Dayer dans Pharmacologie, "Des concepts fondamentaux aux applications thérapeutiques", 1992, chapitre 37, pages 541-561, 2ième édition, Frison-Roche/Slatkine éditeurs. Il s'agit des acides arylcarboxyliques, comme les dérivés de l'acide salicylique ou les dérivés de l'acide anthranilique, des acides arylalcanoïques, tels que les acides arylacétiques et hétéro-arylacétiques ou les acides arylpropioniques, des acides énoliques, comme les dérivés de la pyrazolone ou les oxicames, et des dérivés non acides, comme par exemple le bufexamac (Merck Index, 11ième édition (M.I.) 1462), la benzydamine (M.I. 1136), l'épirizole (M.I. 3572), la fluproquazone (M.I. 4120) ou la tiaramide (M.I. 9356).

Préférentiellement, la composition selon l'invention comprend un composé diminuant la synthèse, la libération et/ou l'activité d'au moins un médiateur de l'inflammation cutanée en association avec un extrait d'au moins une bactérie filamenteuse non photosynthétique.

Le composé diminuant la synthèse, la libération et/ou l'activité d'au moins un médiateur de l'inflammation est de préférence choisi parmi les antagonistes de substance P et/ou de CGRP, les inhibiteurs de NO-synthase, les antagonistes de bradykinine, les antagonistes de cytokines, les antagonistes d'histamine, les antagonistes du facteur de nécrose tumorale de type $\alpha$ (TNF$\alpha$).

Par exemple, selon l'invention, on peut utiliser un ou plusieurs antagonistes de substance P choisis parmi les peptides, les composés non peptidiques comme ceux comprenant au moins un hétérocycle, les composés azotés comprenant au moins un cycle benzénique, les sels de cations monovalents, divalents et trivalents, les eaux thermales, et leurs mélanges.

On peut utiliser dans l'invention par exemple comme peptide antagoniste de substance P le sendide et le spantide II.

Le sendide correspond à la formule :
Tyr D-Phe Phe D-His Leu Met NH$_2$
dans laquelle :

Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule :
D-NicLys Pro 3-Pal Pro D-Cl$_2$Phe Asn D-Trp Phe D-Trp Leu Nle NH$_2$
dans laquelle :

D-NicLys représente le nicotinate de D-lysine,
Pro représente la proline,
3-Pal représente la 3-pyridyl-alanine,
D-Cl$_2$Phe représente la D-dichlorophénylalanine,
Asn représente l'asparagine,
D-Trp représente le D-tryptophane,

Phe représente la phénylalanine,
Leu représente la leucine,
Nle représente la nor-leucine.

On peut également utiliser dans l'invention, comme peptide antagoniste de substance P, les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés comprenant un hétéroatome lié directement ou indirectement à un cycle benzénique ou contenu dans un hétérocycle. En particulier cet hétéroatome est un atome d'oxygène, d'azote ou de soufre.

Comme composé hétérocyclique, on peut notamment utiliser dans l'invention ceux décrits dans les documents suivants : EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolyl-benzofuranne-carboxamides ou les alcoxy- et/ou aryloxy- tétrazolyl-benzothiophène-carboxamides.

Comme composés comportant un atome d'azote lié directement ou indirectement à un noyau benzénique, on peut citer ceux décrits dans les documents suivants : EP-A-522808 et WO-A-93/01165 et WO-A-93/10073.

Les sels de cations utilisables dans l'invention sont notamment les sels de strontium, de magnésium, de lanthanides de numéro atomique allant de 57 à 71, de cobalt, de nickel, de manganèse, de baryum, d'yttrium, de cuivre, d'étain, de rubidium, de lithium et de zinc.

Ces sels peuvent être par exemple des carbonates, des salicylates, des bicarbonates, des sulfates, des glycérophosphates, des borates, des chlorures, des nitrates, des acétates, des hydroxydes, des persulfates ainsi que des sels d'$\alpha$-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate). De préférence, le sel est choisi parmi le nitrate de strontium, de manganèse, d'yttrium ou de magnésium, le borate de strontium, de manganèse d'yttrium ou de magnésium, le chlorure de strontium, de manganèse ou de magnésium, le sulfate de magnésium, de manganèse ou de strontium. Encore plus préférentiellement, ces sels sont le chlorure ou le nitrate de strontium.

Parmi les eaux thermales utilisables selon l'invention, on peut citer plus particulièrement les eaux thermales du bassin de Vichy, comme celles provenant des sources Célestins, Chomel, Grande-Grille, Hôpital, Lucas et Parc. Préférentiellement, selon l'invention, on utilise l'eau de la source Lucas.

Les antagonistes de substance P peuvent être utilisés seuls ou en mélange.

Par antagoniste du CGRP, on entend tout composé susceptible d'inhiber partiellement, voire totalement, l'effet biologique du CGRP.

Particulièrement, pour qu'une substance soit reconnue comme un antagoniste de CGRP elle doit induire une réponse pharmacologique cohérente (incluant ou non sa fixation au récepteur du CGRP) notamment dans l'un des tests suivants :

+ la substance antagoniste doit diminuer la vasodilatation induite par la capsaïcine et/ou par une stimulation électrique antidromique (appliquée sur un nerf afférent) et/ou
+ la substance antagoniste doit provoquer une inhibition de la libération de CGRP par les fibres nerveuses sensitives et/ou
+ la substance antagoniste doit provoquer une inhibition de la contraction du muscle lisse du vas deferens induite par le CGRP.

Parmi les antagonistes du CGRP connus, on peut citer par exemple le CGRP 8-37 (séquence des acides aminés 8 à 37 de la partie N-terminale du CGRP), ou encore les anticorps anti-CGRP.

Les antagonistes de CGRP peuvent être utilisés seuls ou en mélange.

Le terme NO-synthase recouvre en fait une famille d'enzymes qui, de façon spécifique, assurent la catalyse enzymatique de la L-arginine en citrulline, catalyse au cours de laquelle est produit un médiateur gazeux aux multiples fonc-

EP 0 761 204 B1

tions, le monoxyde d'azote ou NO. Le monoxyde d'azote possède de par sa structure un électron supplémentaire le rendant extrêmement réactif chimiquement. Il est notoire que de tels composés sont nocifs et l'on cherche à limiter au mieux leur production. C'est ainsi que dans le cas du monoxyde d'azote les inhibiteurs de NO-synthase ont été largement étudiés.

Ainsi, selon l'invention les inhibiteurs de NO-synthase sont des produits qui permettent *in situ* sur l'homme d'inhiber partiellement, voire totalement, la synthèse de monoxyde d'azote (NO).

Ce sont donc des composés choisis parmi les composés inhibant la synthèse et/ou accélérant le catabolisme de la NO-synthase, les composés neutralisant la NO-synthase ou les composés intervenant en diminuant le signal transduit par la NO-synthase.

Ainsi, l'inhibiteur de la NO-synthase peut être choisi parmi des peptides, synthétiques ou naturels, éventuellement modifiés, des molécules chimiques, synthétiques ou naturelles, des acides nucléiques antisens, des ribozymes, des anticorps anti-NO-synthase.

Parmi ces inhibiteurs de la NO-synthase, on peut citer notamment la $N^G$-monométhyl-L-arginine (L-NMMA), la $N^G$-nitro-L-arginine, l'ester méthylé de la $N^G$-nitro-L-arginine, le chlorure de diphénylèneiodonium, la 7-nitroindazole, la N(5)-(1-iminoéthyl)-L-ornithine, la $N^G,N^G$-diméthyl-L-arginine, la $N^G,N^G$-diméthyl-arginine, le 2-(4-carboxyphényl)-4,4,5,5-tetraméthylimidazoline-1-oxyl-3-oxyde, l'aminoguanidine, la canavanine, l'ebselen et l'hormone stimulatrice des mélanocytes de type $\alpha$.

Parmi les inhibiteurs de la NO-synthase, on utilise préférentiellement la $N^G$-monométhyl-L-arginine et l'hormone stimulatrice des mélanocytes de type $\alpha$. Les inhibiteurs de la NO-synthase peuvent être utilisés seuls ou en mélange.

La bradykinine est un peptide d'origine plasmatique libéré à partir d'un précurseur kininogène par une protéase plasmatique du nom de Kallikreine (EC 3.4.21.24). Ce nanopeptide est un des médiateurs clés de l'inflammation et possède des propriétés mitogènes. Les récepteurs pour cette kinine se divisent en deux principaux sous types, B1 et B2. La bradykinine agit notamment, sur le récepteur B2 et provoque la stimulation de nombreux systèmes de production de seconds messagers dont l'hydrolyse des inositol-phosphates, du métabolisme de l'acide arachidonique, la phosphorylation des résidus tyrosine ainsi que la dépolarisation ou l'hyperpolarisation de la membrane cellulaire. L'activation de certains récepteurs provoque l'activation de la phospholipase C et donc la production de l'inositol 1,4,5-triphosphate (IP3) et de diacylglycérol (DAG). L'IP3 est connu pour provoquer la libération de calcium à partir des sites de stockages intracellulaires dans les cellules dont le kératinocyte. Le calcium, décrit comme activateur et régulateur de nombreuses enzymes (protéases, phospholipases), joue un rôle important dans la régulation de la différenciation et de la prolifération du kératinocyte.

Par antagoniste de la bradykinine, on entend tout composé susceptible d'inhiber partiellement, voire totalement, l'effet biologique de la bradykinine.

Particulièrement, pour qu'une substance soit reconnue comme un antagoniste de la bradykinine elle doit induire une réponse pharmacologique cohérente incluant ou non sa fixation au récepteur de la bradykinine.

Ainsi, entre dans cette définition tout composé qui peut interférer avec les effets de la bradykinine par sa fixation au récepteur de celle-ci (B1 ou B2) et/ou tout composé qui indépendamment de la fixation au(x) récepteur(s) induit par un mécanisme quelconque un effet contraire à celui connu de la bradykinine (par exemple interférant avec la synthèse de la bradykinine).

Parmi les antagonistes de la bradykinine, on préfère utiliser des composés inhibant la synthèse et/ou accélérant le catabolisme de la bradykinine, des composés neutralisant la bradykinine, des composés bloquant les récepteurs de la bradykinine tels que ceux qui interférent avec les effets de la bradykinine par leur fixation au récepteur de celle-ci (B1 ou B2), des composés inhibant la synthèse des récepteurs de la bradykinine ou des composés intervenant en diminuant le signal transduit par la bradykinine. Ces composés peuvent être d'origine naturelle ou synthétique.

Parmi les antagonistes de la bradykinine, on peut citer plus particulièrement des peptides, synthétiques ou naturels, éventuellement modifiés, comme la D-Arg, [Hyp3, D-Phe7]-bradykinin (NPC567), la [Thi 5, 8, D-Phe7]-bradykinin, la D-Arg, [Hyp3, Thi5,8, D-Phe7]-bradykinin, la N-$\alpha$-adamantaneacetyl-D-Arg, [Hyp3, Thi5,8, D-Phe7]-bradykinin, la des-Arg9, [Leu8]-bradykinin (tous vendus par la société Sigma) ou encore les composés cités dans les brevets WO 95/08566, WO 95/07294, EP0623350, EP 0622361, WO 94/11021, EP 0596406, WO 94/06453, WO 94/09001, EP 0578521, EP 0564972, EP 0552106, WO 93/11789, US 5216165, US 5212182, WO 92/17201, EP 0496369, EP 0472220, EP 0455133, WO 91/09055, WO 91/02746, EP 0413277, EP 0370453, EP 0359310, WO 90/03980, WO 89/09231, WO 89/09230, WO 89/01780, EP 0334244, EP 0596406, WO 86/07263 ou la P-guanidobenzoyl, [Hyp3,Thi5,D-Tic7,Oic8]-bradyklnin (S 16118) (Feletou M & al., Pharmacol. Exp. Ther., June 1995, 273, 1078-84), la D-Arg, [Hyp3, Thi5, D-Tic7,Oic8]-bradykinine (HOE 140) (Feletou M & al., Eur. J. Pharmacol. 1995, 274, 57-64), la D-Arg, [Hyp3, D-Hype (trans-propyl) 7, Oic 8]-bradykinin (NPC 17731) ( Herzig M.C.S. and Leeb-Lundberg L.M.F., J. Biol. Chem. 1995, 270, 20591-20598) ou ceux cités dans Bradykinin Antagonists : development and applications (Stewart J.M., Biopolymers, 1995, 37, 143-155), ou encore des molécules chimiques, synthétiques ou naturelles, comme par exemple celles décrites dans Salvino et coll. J. Med. Chem., 1993, 36, 2583-2584.

On peut également utiliser selon l'invention des acides nucléiques antisens ou des ribozymes ayant pour but de sélectivement inhiber la synthèse de la bradykinine. Ces acides nucléiques antisens sont connus de l'homme du métier. Ils peuvent agir de différentes manières sur l'ADN ou sur l'ARN messager codant pour la bradykinine, notamment par blocage de la fixation ou de la progression des ribosomes le long de l'ARN messager, par clivage de l'ARN messager par la RNase H, ou en empêchant le transport de l'ARN messager du noyau vers le cytoplasme ou encore en empêchant la maturation de l'ARN messager.

On peut encore utiliser selon l'invention des anticorps anti-bradykinine ou des récepteurs solubles de la bradykinine, des anticorps anti-récepteurs de la bradykinine ou des antagonistes des récepteurs de la bradykinine.

Préférentiellement, selon l'invention on utilise un composé qui interfère avec les effets de la bradykinine par sa fixation au récepteur de celle-ci (B1 ou B2), préférentiellement au récepteur B2.

Encore plus préférentiellement, on utilise selon l'invention un antagoniste de la bradykinine choisi parmi :

la D-Arg, [Hyp3, D-Phe7]-bradykinin (NPC567),
la [Thi 5, 8, D-Phe7]-bradykinin, la D-Arg, [Hyp3, Thi5,8, D-Phe7]-bradykinin,
la N-$\alpha$-adamantaneacetyl-D-Arg, [Hyp3, Thi5,8, D-Phe7]-bradykinin,
la des-Arg9, [Leu8]-bradykinin,
la P-guanidobenzoyl, [Hyp3,Thi5,D-Tic7,Oic8]-bradyklnin (S 16118),
la D-Arg, [Hyp3, Thi5, D-Tic7,Oic8]-bradykinine (HOE 140),
la D-Arg, [Hyp3, D-Hype (trans-propyl) 7, Oic 8]-bradykinin (NPC 17731)

Le peptide modifié préférentiellement utilisé selon l'invention est la D-Arg, [Hyp3, Thi5, D-Tic7,Oic8]-bradykinine (HOE 140).

Les antagonistes de bradykinine peuvent être utilisés seuls ou en mélange.

On sait, par ailleurs, que la substance P libérée par les terminaisons sensitives épidermiques induit une cascade d'événements biochimiques dont les premières étapes se situent au niveau des mastocytes. La fixation de la substance P sur les récepteurs mastocytaires induit une libération de nombreux médiateurs pro-inflammatoires parmi lesquels l'histamine, les cytokines comme l'interleukine 1 (IL1), l'interleukine 6 (IL6), l'interleukine 8 (IL8) et le facteur de nécrose tumorale de type $\alpha$ (Tumor Necrosis Factor $\alpha$ (TNF-$\alpha$)).

On entend par antagonistes d'histamine, de cytokines et/ou de TNF-$\alpha$, toutes substances susceptibles d'inhiber la libération et/ou la synthèse et/ou la fixation réceptorielle respectivement d'histamine, de cytokines et/ou de TNF-$\alpha$.

Les antagonistes inhibant la fixation réceptorielle de l'histamine sont des agents spécifiques du récepteur de type 1 de l'histamine (H1).

Pour qu'une substance soit reconnue comme un antagoniste réceptoriel d'histamine, de cytokines ou de TNF-$\alpha$, elle doit répondre à l'une des caractéristiques suivantes :

- avoir une affinité pour les récepteurs spécifiques de ces composés ;
- avoir une activité pharmacologique antagoniste réceptoriel d'histamine, de cytokines ou de TNF-$\alpha$, c'est-à-dire induire une réponse pharmacologique cohérente dans l'un des tests suivants :

  + pour les antagonistes réceptoriels d'histamine : une inhibition de la contraction des muscles lisses induite par l'administration d'histamine ;
  + pour les antagonistes réceptoriels de cytokines: inhibition de l'adhésion de macrophages induite par les cytokines sur les cellules endothéliales ou inhibition de la libération d'anions superoxydes induite par les cytokines sur les neutrophiles ;
  + pour les antagonistes réceptoriels de TNF-$\alpha$ : inhibition de l'adhésion de macrophages induite par TNF-$\alpha$ sur les cellules endothéliales ou inhibition de la libération d'anions superoxydes induite par TNF-$\alpha$ sur les neutrophiles ou inhibition de l'activité mitogène du TNF-$\alpha$ sur les fibroblastes du derme.

Pour qu'une substance soit reconnue comme un antagoniste de la libération et/ou de la synthèse d'histamine, de cytokines ou de TNF-$\alpha$, elle doit répondre à l'une des caractéristiques suivantes :

- inhibition de la libération d'histamine par des mastocytes stimulés par le composé 48/80 ou stimulés par un ionophore calcique (A23 187)
- inhibition de la libération de cytokines ou de TNF-$\alpha$ par des monocytes (cellules U937) différenciés par un ester de phorbol (PMA).

Les antagonistes réceptoriels d'histamine H1 utilisables dans l'invention sont ceux classiquement utilisés dans les traitements des états allergiques et anaphylactiques ainsi que ceux pour lutter contre le mal des transports. Ces composés peuvent être par exemple des dérivés de la diéthylène diamine comme la Cinnarizine, la cyclizine ; des dérivés de l'aminopropane comme la dexchlorophéniramine, la triprolidine ; des dérivés de phénothiazine comme la prométhazine, l'alimémazine ; ainsi que les composés cités pages 116 à 118 du livre Joseph R. Prous, The Year's Drug News, Therapeutic Targets, édition 1994, Prous Science Publishers comme la cétirizine HCl, l'ébastine, la loratadine, la sétastine HCl.

Les inhibiteurs de libération de l'histamine sont notamment des composés hétérocycliques oxygénés ou soufrés tels que les dérivés du furanne, les dérivés du benzofuranne, les dérivés du thiophène et les dérivés du benzothiophène, comportant éventuellement des substituants azotés, tels que ceux décrits dans les documents US-A-4931459, US-A-4910317 et EP-A-299457, et plus spécialement les alcoxy- et/ou aryloxy- tétrazol-yl-benzofuranne-carboxamides ou les alcoxy-et/ou aryloxy- tétrazol-yl-benzothiophène-carboxamides. A titre d'exemple, on peut citer le 5-méthoxy-3-phénoxy-N-1H-tétrazol-5-yl-benzothiophène-2-carboxamide, le 5-méthoxy-3-(1-méthyléthoxy)-N-1H-tétrazol-5-yl-benzothiophène-2-carboxamide, le 6-méthoxy-3-(1-méthyléthoxy)-N-1H-tétrazol-5-yl-benzothiophène-2-carboxamide, le 5-méthoxy-3-(1-méthyléthyl)-N-1H-tétrazol-5-yl-benzothiophène-2-carboxamide, le 3-benzyloxy-5-méthoxy-N-1H-tétrazol-5-yl-benzothiophène-2-carboxamide et le 5-méthoxy-3-phénoxy-N-1H-tétrazol-5-yl-benzothiophène-2-carboxamide.

Parmi les antagonistes de cytokines, on citera par exemple un antagoniste de la libération de l'interleukine 1 utilisable dans l'invention qui peut être l'auranofine ou le SKF-105809 ou encore un antagoniste de la synthèse d'interleukine 1 qui peut être la lactoférrine.

Les antagonistes réceptoriels de TNF-$\alpha$ et les inhibiteurs de la libération et/ou de la synthèse de TNF-$\alpha$ utilisables dans l'invention sont en particulier la lisophyline, l'A802715, la sulfasalazine.

Les antagonistes d'histamine, de cytokines et de TNF-$\alpha$ peuvent être synthétisés ou extraits de produits naturels (végétaux ou animaux).

Les antagonistes d'histamine, de cytokines et de TNF-$\alpha$ peuvent être utilisés selon l'invention, séparément ou associés, seuls ou sous forme de mélange.

La quantité de composé diminuant la synthèse, la libération et/ou l'activité d'au moins un médiateur de l'inflammation contenue dans la composition de l'invention est bien entendu fonction de l'effet recherché et peut donc varier dans une large mesure.

Pour donner un ordre de grandeur, la composition cosmétique de l'invention peut contenir un composé diminuant la synthèse, la libération et/ou l'activité d'au moins un médiateur de l'inflammation en une quantité représentant de 0,0001% à 5% du poids total de la composition et préférentiellement en une quantité représentant de 0,001% à 2% du poids total de la composition.

Pour donner un ordre de grandeur, la composition pharmaceutique de l'invention peut contenir un composé diminuant la synthèse, la libération et/ou l'activité d'au moins un médiateur de l'inflammation en une quantité représentant de 0,0001% à 20% du poids total de la composition et préférentiellement en une quantité représentant de 0,01% à 5% du poids total de la composition.

Quelque soit la forme de la composition selon l'invention, la quantité d'extrait d'au moins une bactérie filamenteuse non photosynthétique contenue dans la composition de l'invention est bien entendu fonction de l'effet recherché. Elle dépend également de la forme de l'extrait utilisé dans la composition (extrait aqueux, lyophilisé, ...). Elle peut donc varier dans une large mesure.

Pour donner un ordre de grandeur, si la composition est une composition cosmétique elle peut contenir un extrait d'au moins une bactérie filamenteuse non photosynthétique en une quantité représentant de 0,0001% à 5% du poids total de la composition et préférentiellement en une quantité représentant de 0,001% à 1% du poids total de la composition.

Pour donner un ordre de grandeur, si la composition est une composition pharmaceutique elle peut contenir un extrait d'au moins une bactérie filamenteuse non photosynthétique en une quantité représentant de 0,0001% à 10% du poids total de la composition et préférentiellement en une quantité représentant de 0,01% à 5% du poids total de la composition.

L'extrait bactérien peut être utilisé dans une composition qui doit être ingérée, injectée ou appliquée sur la peau (sur toute zone cutanée du corps), les cheveux, les ongles ou les muqueuses (buccale, jugale, gingivale, génitale, anale, conjonctive). Selon le mode d'administration, cette composition peut se présenter sous toutes les formes galéniques normalement utilisées.

Pour une application topique sur la peau, la composition peut avoir la forme notamment de solution aqueuse ou suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydre, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, de suspension huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosacée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

L'extrait bactérien utilisé selon l'invention peut aussi être incorporé dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, des shampooings antiparasitaires, etc.

Les compositions peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose$^R$ 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention on peut, entre autres, associer au moins un extrait d'au moins une bactérie filamenteuse non photosynthétique à d'autres agents actifs destinés notamment à la prévention et/ou au traitement des affections cutanées.

Parmi ces agents actifs, on peut citer à titre d'exemple :

- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Ainsi, selon un mode particulier, l'invention concerne l'utilisation d'au moins un extrait d'au moins une bactérie filamenteuse non photosynthétique dans une composition comprenant au moins un agent choisi parmi les agents antibactériens, antiparasitaires, antifongiques, antiviraux anti-inflammatoires, antiprurigineux, anesthésiques, anti-radicaux libres, anti-séborrhéiques, antipelliculaires, antiacnéiques et/ou les agents modulant la différenciation et/ou la prolifération et/ou la pigmentation cutanée.

La présente invention a en outre pour objet un procédé de traitement cosmétique en vue de diminuer l'effet irritant d'une composition cosmétique, caractérisé par le fait que l'on applique sur la peau, sur les cheveux, et/ou sur les muqueuses, une composition telle que décrite ci-dessus.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention sans la limiter aucunement. Dans les compositions les proportions indiquées sont des pourcentages en poids

Exemple 1 : Préparation d'un extrait de _Vitreoscilla filiformis_ :

La souche de _Vitreoscilla filiformis_ (ATCC 15551) est mise en culture selon le procédé décrit dans la demande de brevet WO-A-93-00741. La culture s'effectue à 26°C durant au moins 48 heures jusqu'à l'obtention d'une concentration cellulaire convenable correspondant à une densité optique à 600 nm supérieure ou égale à 1,5. On repique la souche à 2 % V/V dans du milieu neuf toutes les 48 heures jusqu'à l'obtention d'une culture stable. Un Erlenmeyer de 1 litre contenant 200 ml de milieu neuf est alors ensemencé avec 4 ml de la culture précédente.

La culture en Erlenmeyer s'effectue à 26° C sur une table de culture agitée à 100 tours/minutes. Le pied de cuve ainsi obtenu sert d'inoculum à un fermenteur de 10 l. La croissance s'effectue à 26° C, pH 7, 100 tours/minute et $pO_2 \geq 15$ %. Après 48 heures de croissance, la biomasse est transférée dans un fermenteur de 600 litres utiles, pour être cultivée dans les mêmes conditions. Après 48 H de croissance on récolte les cellules. La biomasse est alors concentrée 50 fois environ par centrifugation.

Le concentré est autoclavé à 121° C durant 40 minutes. Après refroidissement 2 phases apparaissent. La phase liquide surnageante est alors filtrée à 0,22 µm pour éliminer les particules. Cet extrait est utilisable en l'état (forme aqueuse) ou peut être lyophilisé suivant les techniques classiques (forme lyophilisée).

Exemple 2 : Mesure de l'affinité réceptorielle de l'extrait bactérien (exemple 1, forme aqueuse) pour le récepteur NK1 humain (récepteur à la substance P humain) :

A) : Affinité réceptorielle :

La mesure de l'affinité réceptorielle de l'extrait bactérien pour le récepteur NK1 humain a été effectuée selon la méthode décrite dans l'article : Heuillet, E. et al, J. Neurochem. ,60, 1993, 868-876.

L'extrait est testé aux concentrations de 1 %, 5 % et 10 %.

Lors de chaque expérience, la molécule de référence du récepteur étudié ([Sar[9], Met $(O_2)$[11]]-SP, analogue de la substance P décrit par Heuillet, E. (Heuillet, E. et al, J. Neurochem. ,60, 1993, 868-876)) est parallèlement testée à 8 concentrations (n = 2) pour l'obtention d'une courbe standard permettant de valider l'expérimentation.

On obtient ainsi :

9 % de fixation pour l'extrait de l'exemple 1 à 1%
27 % de fixation pour l'extrait de l'exemple 1 à 5 %
91 % de fixation pour l'extrait de l'exemple 1 à 10 %

Les résultats de cette expérience mettent en évidence une affinité de l'extrait bactérien sous forme aqueuse pour le récepteur à la substance P humain dès la concentration de 1%.

La courbe d'affinité tracée d'après les résultats obtenus montre 50% de déplacement du ligand naturel (IC50) par l'extrait bactérien à la concentration de 6,7%.

B) : Test fonctionnel *in vitro* :

Un test fonctionnel *in vitro* réalisé sur les récepteurs NK1 humains (récepteurs à la substance P humain) présents sur les muscles lisses d'intestin isolé (iléon) est réalisé pour mettre en évidence le caractère antagoniste de substance P de l'extrait bactérien.

Les expériences *in vitro* sont réalisées selon la méthode décrite par Dion et al. (Life Sciences, 41, 1987, 2269-2278) et Patacchini et al. (Eur. J. Pharmacol., 215, 1992, 93-98).

Après installation dans des cuves expérimentales, les tissus (muscles lisses) sont soumis à une tension initiale de 1 g. Une période d'équilibration d'au moins 60 minutes, au cours de laquelle la solution physiologique est plusieurs fois renouvelée et la tension initiale réajustée, est ensuite respectée avant l'addition de l'extrait.

Les expériences sont conduites en présence continue d'atropine ($3.10^{-6}$ M), de pyrilamine ($3.10^{-6}$ M) et d'indométhacine ($10^{-6}$ M) pour s'affranchir des effets indirects de médiateurs mis en jeu lors de la stimulation d'autres types de récepteurs présents sur ce tissu.

Chaque préparation est initialement stimulée par un agoniste de substance P : [Sar[9], Met $(O_2)$[11]]-SP, à la concentration de $10^{-8}$ M pour l'obtention d'une réponse contractile "contrôle", puis la solution physiologique est entièrement renouvelée.

Cette opération est ensuite répétée toutes les 40 minutes en présence de concentrations croissantes de l'extrait bactérien, chacune d'elles étant ajoutée 30 minutes avant la [Sar[9], Met $(O_2)$[11]]-SP.

Une inhibition de 50 % de l'activité de la [Sar[9], Met $(O_2)$[11]]-SP est obtenue à la concentration de 5 % en extrait.

C) : Test fonctionnel *in vivo* :

Un test fonctionnel *in vivo* est réalisé sur un modèle d'inflammation neurogène pour mettre en évidence le caractère antagoniste de substance P de l'extrait bactérien.

Les expériences *in vivo* sont réalisées selon la méthode décrite par Xu X.J. et collaborateurs (Neurosciences, 1991, 42, 731-737).

Le test consiste à provoquer une inflammation neurogène par la stimulation antidromique du nerf saphène chez l'animal anesthésié. Ce nerf innerve les territoires cutanés des pattes postérieures.

La stimulation provoque la libération à partir des terminaisons nerveuses de substance P, responsable en partie de l'inflammation neurogène.

L'inflammation neurogène est quantifiée par la mesure de la perméabilité tissulaire au bleu Evans, un marqueur de l'extravasation tissulaire de l'albumine plasmatique qui a lieu au cours de l'inflammation.

Ce modèle de référence est utilisé pour la recherche *in vivo* d'antagonistes de la substance P.

L'extrait bactérien sous sa forme aqueuse, administré dilué au 1/100 ème, provoque une diminution statistiquement significative de 51 % de l'inflammation neurogène.

Conclusions :

L'extrait bactérien présente une affinité pour le récepteur de la substance P et exerce une activité antagoniste de la substance P.

Exemple 3 : Evaluation de l'effet apaisant d'un extrait de *Vitreoscilla filiformis* :

A) : Protocole :

1) Sélection des sujets

25 sujets à peau sensible ont été sélectionnés par un dermatologue selon les critères du test à l'acide lactique (K. Lammintausta et al., Dermatoses, 1988, 36, pages 45-49 ; T. Agner et J. Serup, Clinical and Experimental Dermatology, 1989, 14, pages 214-217).

Les sujets retenus sont des sujets présentant une réactivité modérée et des sujets présentant une réactivité forte.

2) Déroulement du test

Un traitement complet est effectué par chaque sujet. Ce traitement est réalisé deux fois par jour et comprend successivement :

- une phase de nettoyage effectuée suivant l'habitude du sujet à l'aide

    + d'une crème nettoyante (formule A) dans le cas de l'utilisation de l'eau pour le nettoyage de la peau ;
    + d'un lait de nettoyage (formule B) lors du nettoyage sans eau ;

- l'utilisation d'une lotion de soin (formule C) appliquée après le nettoyage sur une peau parfaitement sèche ;
- l'utilisation d'une crème de soin (formule D) appliquée après la lotion.

Le test s'est déroulé sur une période de 4 semaines avec contrôle de la réactivité de la peau :

.avant la première application des produits,   T1
.après 7 jours de traitement,   T8
.après 2 semaines de traitement,   T15
.après 4 semaines de traitement,   T29

Le contrôle est effectué selon le procédure suivante :
L'application se fait en simple aveugle.

- application sur un sillon naso-génien d'un coton imbibé de 0,1 ml d'une solution aqueuse à 10 % d'acide lactique. Ce coton est passé 10 fois de suite dans le sillon naso-génien.
- application sur le sillon naso-génien contra-latéral d'un coton imbibé de 0,1 ml sérum physiologique. Ce coton est passé 10 fois de suite dans le sillon naso-génien.
- évaluation de la sensation de picotement sur chaque sillon naso-génien par le sujet lui-même au cours des 30 premières secondes, à 2 et 5 minutes en attribuant une note selon l'échelle suivante :

0   = pas de picotement
1   = picotement léger
2   = picotement modéré
3   = picotement sévère.

Le score est établi en faisant la différence des sommes des scores totaux (30 sec., 2 et 5 mn) pour chaque sillon entre le côté traité par l'acide lactique et l'autre côté traité par le sérum physiologique, soit :

Score = (somme scores acide lactique) - ( somme scores sérum physiologique).

B) : Compositions utilisées pour le test :

| (Formule A) : Crème de nettoyage | |
|---|---|
| Extrait de l'exemple 1 (sous sa forme lyophilisée) | 0.05 |
| Alcool Cétylique | 2.00 |
| Stéarate de Glycérol | 2.00 |
| Acide Stéarique | 2.00 |
| Polyglyceryl-3 Hydroxylauryl Ether | 5.00 |
| Huile Minérale Codex | 12.00 |
| Carbomer | 0.35 |
| Hydroxyde de Sodium | 0.15 |
| Parfum          qsp | |
| Méthylparaben | 0.20 |
| Eau déminéralisée Stérile          qsp | 100.00 |

| (Formule B) Lait de nettoyage | |
|---|---|
| Extrait de l'exemple 1 (sous sa forme lyophilisée) | 0.05 |
| Carbomer | 0.40 |
| Hydroxyde de Sodium | 0.10 |
| Huile Minérale Codex | 5.00 |
| Stéarate de Glycérol | 1.00 |
| Alcool Cétylique | 0.50 |
| PEG 100 Stéarate | 0.80 |
| Méthylparaben | 0.20 |
| Parfum          qsp | |
| Eau déminéralisée Stérile          qsp | 100.00 |

| (Formule C) Lotion de soin | |
|---|---|
| Extrait de l'exemple 1 (sous sa forme lyophilisée) | 0.05 |
| Glycérol | 2.00 |
| Méthylparaben | 0.15 |
| Parfum          qsp | |
| Eau déminéralisée Stérile          qsp | 100.00 |

| (Formule D) Crème de soin | |
|---|---|
| Extrait de l'exemple 1 (sous sa forme lyophilisée) | 0.05 |
| Stéarate de Glycérol | 1.00 |
| PEG 100 Stéarate | 1.00 |
| Acide Stéarique | 1.00 |
| Alcool Cétylique | 2.00 |
| Huile de Soja | 3.00 |
| Huile de Palme | 2.00 |
| Cyclométhicone | 2.00 |
| Diméthicone | 1.00 |
| Polyacrylamide | 0.20 |
| Glycérol | 3.00 |
| Méthylparaben | 0.20 |
| Parfum          qsp | |
| Eau Stérile Déminéralisée          qsp | 100.00 |

C) : Résultats (en unités arbitraires) :

| N° sujet | T1 | T8 | T15 | T29 |
|---|---|---|---|---|
| 1 | 2 | 8 | 5 | - |
| 2 | 6 | 1 | 2 | 1 |
| 3 | 8 | 2 | 5 | 3 |
| 4 | 8 | 4 | 2 | 5 |
| 5 | 3 | 4 | 3 | 1 |
| 6 | 8 | nf | nf | 1 |
| 7 | 5 | 4 | 0 | 2 |
| 8 | 5 | 6 | 6 | 8 |
| 9 | 8 | 4 | 1 | 0 |
| 10 | 6 | 2 | -2 | 2 |
| 11 | 4 | 4 | 3 | 5 |
| 12 | 3 | 3 | 1 | 3 |
| 13 | 5 | 0 | 1 | 4 |
| 14 | 4 | 0 | 0 | 0 |
| 15 | 2 | 2 | 1 | 2 |
| 16 | 2 | 1 | 1 | 2 |
| 17 | 2 | 0 | 3 | -1 |
| 18 | 3 | 4 | 3 | -2 |
| 19 | 7 | 0 | 0 | 3 |
| 20 | 6 | 5 | 3 | 2 |
| 21 | 6 | 4 | 3 | 4 |
| 22 | 3 | 6 | 2 | 3 |
| 23 | 3 | 0 | 0 | -1 |
| 24 | 6 | 1 | 5 | 2 |
| 25 | 6 | 1 | 0 | 3 |
| | | | | |
| TOTAL | 121 | 66 | 48 | 52 |
| MOYENNE | 4.84 | 2.75 | 2.00 | 2.17 |
| ECART TYPE | 2.14 | 2.29 | 1.91 | 2.29 |
| % EVOLUTION | | - 43.2 | - 58.7 | - 55.2 |
| nf: non fourni. | | | | |

Il est constaté dès la première semaine de traitement une très forte diminution de la sensibilité cutanée, diminution qui se poursuit jusqu'à l'issue du traitement.

Exemple 4: Préparation d'un extrait d'Iris pallida :

Des cellules indifférenciées d'Iris pallida cultivées *in vitro* en conditions axéniques sont récupérées après culture en Erlenmeyer ou en fermenteur par filtration sur tamis de 50μm. A 55 g de matière fraîche ainsi obtenue, on ajoute 27,5 ml d'eau déminéralisée. L'ensemble est broyé (Potter, Ultra Turrax...) au Turrax à 24000 T/min durant 1 minute à 4°C (bain de glace). Le broyat est centrifugé 15 à 10000 G à 4°C. Le surnageant est filtré à 0,22 μm (filtration stérilisante).

L'extrait ainsi préparé est conservé à 4°C. Il renferme environ 15 g de matière sèche par litre.

Si le matériel végétal est de la plante entière, on ramène la matière fraîche à traiter en fonction du poids sec pour se mettre dans les mêmes conditions d'extraction que pour l'*in vitro*. Les différentes parties de la plante sont prélevées en fonction du poids relatif de chaque partie de celle-ci. Le traitement à froid permet de geler les activités enzymatiques, la filtration stérilisante évite la dégradation des actifs par les micro-organismes de l'environnement. Enfin, le véhicule eau est compatible avec les récepteurs *ex vivo* et facilite les formulations cosmétiques ou pharmaceutiques.

Exemples 5:

Exemples de formulations illustrant l'invention et particulièrement les compositions selon l'invention associant au moins un extrait d'au moins une bactérie filamenteuse non photosynthétique et un produit à effet irritant. Ces compositions ont été obtenues par simple mélange des différents composants.

| Composition 1 : Lotion démaquillante pour le visage | |
|---|---|
| Extrait de l'exemple 1 (sous sa forme lyophilisée) | 0,01 |
| Antioxydant | 0,05 |
| Isopropanol | 40,00 |
| Conservateur | 0,30 |
| Eau          qsp | 100 % |

| Composition 2 : Gel pour le soin du visage | |
|---|---|
| Extrait de l'exemple 1 (sous sa forme lyophilisée) | 0,05 |
| Hydroxypropylcellulose (Klucel H vendu par la société Hercules) | 1,00 |
| Antioxydant | 0,05 |
| Isopropanol | 40,00 |
| Conservateur | 0,30 |
| Eau          qsp | 100 % |

| Composition 3 : Crème de soin du visage (émulsion huile dans eau) | |
|---|---|
| Extrait de l'exemple 1 (sous sa forme aqueuse) | 2,00 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau       qsp | 100 % |

| Composition 4 : Shampooing | |
|---|---|
| Extrait de l'exemple 1 (sous sa forme aqueuse) | 1,00 |
| Hydroxypropylcellulose (Klucel H vendu par la société Hercules) | 1,00 |
| Parfum | 0,50 |
| Conservateur | 0,30 |
| Eau       qsp | 100 % |

| Composition 5 : Crème de soin antirides pour le visage (émulsion huile/eau) | |
|---|---|
| Extrait de l'exemple 1 (sous sa forme lyophilisée) | 0,05 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau          qsp | 100 % |

| Composition 6: Gel anti-douleur | |
|---|---|
| Extrait de l'exemple 1 (sous sa forme lyophilisée) | 0,03 |
| Hydroxypropylcellulose (Klucel H vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau          qsp | 100 % |

| Composition 7 : Crème de soin de l'érythème solaire (émulsion huile-dans-eau) ||
|---|---|
| Extrait de l'exemple 1 (sous sa forme aqueuse) | 0,75 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,50 |
| Conservateur | 0,30 |
| Eau          qsp | 100 % |

| Composition 8 : Gel pour le traitement de l'acné ||
|---|---|
| Extrait de l'exemple 1 (sous sa forme aqueuse) | 0,50 |
| Acide tout trans rétinoïque | 0,05 |
| Hydroxypropylcellulose (Klucel H) | 1,00 |
| Antioxydant | 0,05 |
| Isopropanol | 40,00 |
| Conservateur | 0,30 |
| Eau          qsp | 100 % |

| Composition 9 : Lotion pour éliminer les cicatrices dues à l'acné ||
|---|---|
| Extrait de l'exemple 1 (sous sa forme lyophilisée) | 0,025 |
| Acide glycolique | 50,00 |
| Hydroxypropylcellulose (Klucel H) | 0,05 |
| NaOH          qsp | pH =2,80 |
| Ethanol          qsp | 100 % |
| Conservateur | 0,30 |

| Composition 10: Gel pour le soin du visage | |
|---|---|
| Extrait de l'exemple 1 | 10,00 |
| Extrait de l'exemple 4 | 0,50 |
| Hydroxypropylcellulose (Klucel H vendu par la société Hercules) | 1,00 |
| Antioxydant | 0,05 |
| Isopropanol | 40,00 |
| Conservateur | 0,30 |
| Eau          qsp | 100 % |

| Composition 11 : Lotion démaquillante pour le visage | |
|---|---|
| Extrait de l'exemple 1 | 5,00 |
| Extrait de l'exemple 4 | 0,10 |
| Isopropanol | 40,00 |
| Conservateur | 0,30 |
| Eau          qsp | 100 % |

| Composition 12 : Crème de soin du visage (émulsion huile dans eau) | |
|---|---|
| Extrait de l'exemple 1 | 7,00 |
| Extrait de l'exemple 4 | 1,00 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Conservateur | 0,30 |
| Eau          qsp | 100 % |

| Composition 13 : Shampooing | |
|---|---|
| Extrait de l'exemple 1 | 2,00 |
| Extrait de l'exemple 4 | 0,50 |
| Hydroxypropylcellulose (Klucel H vendu par la société Hercules) | 1,00 |
| Parfum | 0,50 |
| Conservateur | 0,30 |
| Eau          qsp | 100 % |

| Composition 14 : Lotion pour éliminer les cicatrices dues à l'acné | |
|---|---|
| Extrait de l'exemple 1 | 8,00 |
| Extrait de l'exemple 4 | 1,00 |
| Acide glycolique | 50,00 |
| Hydroxypropylcellulose (Klucel H vendu par la société Hercules) | 0,05 |
| NaOH          qsp | pH = 2,8 |
| Ethanol          qsp | 100 % |
| Conservateur | 0,30 |

| Composition 15 : Gel pour le traitement de l'acné | |
|---|---|
| Extrait de l'exemple 1 | 8,00 |
| Extrait de l'exemple 4 | 1,00 |
| Acide tout trans rétinoïque | 0,05 |
| Hydroxypropylcellulose (Klucel H vendu par la société Hercules) | 1,00 |
| Antioxydant | 0,05 |
| Isopropanol | 40,00 |
| Conservateur | 0,30 |
| Eau          qsp | 100 % |

| Composition 16 : Gel anti-douleur | |
|---|---|
| Extrait de l'exemple 1 | 0,50 |
| Extrait de l'exemple 4 | 10,00 |
| Hydroxypropylcellulose (Klucel H vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau          qsp | 100 % |

| Composition 17 : Crème de soin de l'érythème solaire (émulsion huile-dans-eau) | |
|---|---|
| Extrait de l'exemple 1 | 5,00 |
| Extrait de l'exemple 4 | 1,00 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,50 |
| Conservateur | 0,30 |
| Eau          qsp | 100 % |

| Composition 18 : Crème de soin antirides pour le visage (émulsion huile/eau) | |
|---|---|
| Extrait de l'exemple 1 | 1,00 |
| Extrait de l'exemple 4 | 8,00 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau          qsp | 100 % |

| Composition 19 : Lotion démaquillante pour le visage | |
|---|---|
| Extrait de l'exemple 1 | 1,00 |
| Chlorure de strontium | 5,00 |
| Antioxydant | 0,05 |
| Isopropanol | 40,00 |
| Conservateur | 0,30 |
| Eau          qsp | 100 % |

| Composition 20 : Gel pour le soin du visage | |
|---|---|
| Extrait de l'exemple 1 | 0,50 |
| Eau thermale de Vichy | 10,00 |
| Hydroxypropylcellulose (Klucel H vendu par la société Hercules) | 1,00 |
| Antioxydant | 0,05 |
| Isopropanol | 40,00 |
| Conservateur | 0,30 |
| Eau          qsp | 100 % |

| Composition 21 : Crème de soin du visage (émulsion huile dans eau) | |
|---|---|
| Extrait de l'exemple 1 | 1,00 |
| Auranofine | 0,10 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Conservateur | 0,30 |
| Eau        qsp | 100 % |

| Composition 22 : Shampooing | |
|---|---|
| Extrait de l'exemple 1 | 0,50 |
| Chlorure de strontium | 5,00 |
| Hydroxypropylcellulose (Klucel H vendu par la société Hercules) | 1,00 |
| Conservateur | 0,30 |
| Eau        qsp | 100 % |

| Composition 23 : Lotion pour éliminer les cicatrices dues à l'acné | |
|---|---|
| Extrait de l'exemple 1 | 1,00 |
| HOE 140 | 0,05 |
| Acide glycolique | 50,00 |
| Hydroxypropylcellulose (Klucel H vendu par la société Hercules) | 0,05 |
| NaOH        qsp | pH = 2,8 |
| Ethanol        qsp | 100 % |
| Conservateur | 0,30 |

| Composition 24 : Gel pour le traitement de l'acné | |
|---|---|
| Extrait de l'exemple 1 | 2,00 |
| CGRP 8-37 | 0,50 |
| Acide tout trans rétinoïque | 0,05 |
| Hydroxypropylcellulose (Klucel H vendu par la société Hercules) | 1,00 |
| Antioxydant | 0,05 |
| Isopropanol | 40,00 |
| Conservateur | 0,30 |
| Eau          qsp | 100 % |

| Composition 25 : Gel anti-douleur | |
|---|---|
| Extrait de l'exemple 1 | 0,50 |
| Spantide II | 0,05 |
| Hydroxypropylcellulose (Klucel H vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau          qsp | 100 % |

| Composition 26 : Crème de soin de l'érythème solaire (émulsion huile-dans-eau) | |
|---|---|
| Extrait de l'exemple 1 | 1,00 |
| Eau thermale de Vichy | 10,00 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,50 |
| Conservateur | 0,30 |
| Eau          qsp | 100 % |

| Composition 27 : Crème de soin antirides pour le visage (émulsion huile-eau) | |
|---|---|
| Extrait de l'exemple 1 | 1,00 |
| Lactoférrine | 1,00 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau          qsp | 100 % |

**Revendications**

1.  Utilisation d'au moins un extrait d'au moins une bactérie filamenteuse non photosynthétique comme antagoniste de substance P dans une composition cosmétique ou pour la préparation d'une composition pharmaceutique.

2. Utilisation d'au moins un extrait d'au moins une bactérie filamenteuse non photosynthétique dans une composition cosmétique ou pour la préparation d'une composition pharmaceutique destinée à traiter les désordres associées à un excès de synthèse et/ou de libération de substance P.

3. Utilisation selon l'une quelconque des revendications 1 ou 2, caractérisée en ce que la composition est destinée à traiter les désordres du système nerveux central, les troubles respiratoires, les syndromes allergiques, l'inflammation, la douleur, les désordres gastro-intestinaux, les désordres cutanés, les fibroses, les troubles de la maturation du collagène les troubles cardio-vasculaires, les troubles vasospastiques, les désordres immunologiques et/ou les troubles du tractus urinaires.

4. Utilisation selon l'une quelconque des revendications 1 à 3, caractérisée en ce que la composition cosmétique ou pharmaceutique est destinée à traiter les peaux sensibles.

5. Utilisation selon l'une quelconque des revendications 1 à 4, caractérisée en ce que la composition cosmétique ou pharmaceutique est destinée à prévenir et/ou lutter contre les irritations cutanées et/ou les dartres et/ou les érythèmes et/ou les sensations dysesthésiques et/ou les sensations d'échauffement et/ou les prurits de la peau et/ou les muqueuses.

6. Utilisation selon l'une quelconque des revendications 1 à 5, caractérisée en ce que ladite bactérie appartient à l'ordre des Beggiatoales.

7. Utilisation selon la revendication 6, caractérisée en ce que ladite bactérie appartient au genre Beggiatoa, Vitreoscilla, Flexithrix ou Leucothrix.

8. Utilisation selon la revendication 7, caractérisée en ce que ladite bactérie est choisie parmi des souches de *Vitreoscilla filiformis*.

9. Utilisation dans une composition cosmétique selon l'une quelconque des revendications 1 à 8, caractérisée en ce que ledit extrait bactérien est utilisé en une quantité représentant de 0,0001 % à 20 % du poids total de la composition et de préférence en une quantité représentant de 0,001 % à 10% du poids total de la composition.

10. Utilisation dans une composition pharmaceutique selon l'une quelconque des revendications 1 à 8, caractérisée en ce que ledit extrait bactérien est utilisé en une quantité représentant de 0,0001 % à 30 % du poids total de la composition et de préférence en une quantité représentant de 0,05 % à 20 % du poids total de la composition.

11. Utilisation selon l'une des revendications 1 à 10, caractérisée en ce que la composition cosmétique ou pharmaceutique comprend en outre au moins un produit à effet irritant.

12. Utilisation selon l'une des revendications 1 à 11, caractérisée en ce que la composition cosmétique ou pharmaceutique comprend en outre au moins un extrait de cellules d'au moins un végétal de la famille des Iridacées.

13. Utilisation selon l'une des revendications 1 à 12, caractérisée en ce que la composition cosmétique ou pharmaceutique comprend en outre au moins un composé diminuant la synthèse, la libération et/ou l'activité d'au moins un médiateur de l'inflammation.

14. Composition cosmétique ou pharmaceutique caractérisée en ce qu'elle comprend, dans un milieu cosmétiquement ou pharmaceutiquement acceptable un extrait d'au moins une bactérie filamenteuse non photosynthétique et au moins un produit à effet irritant choisi parmi les solvants organiques ou les actifs comme les $\alpha$-hydroxy-acides, les $\beta$-hydroxy-acides, les $\alpha$-céto-acides, les $\beta$-céto-acides, les rétinoïdes, les anthralines, les anthranoïdes, les peroxydes, le minoxidil, les sels de lithium, les antimétabolites, les kératolytiques, la vitamine D et ses dérivés, les teintures ou colorants capillaires, les solutions alcooliques parfumantes, les agents antitranspirants, les actifs dépilatoires, les actifs de permanentes, les actifs dépigmentants.

15. Composition cosmétique ou pharmaceutique, caractérisée en ce qu'elle comprend, dans un milieu cosmétiquement ou pharmaceutiquement acceptable, un extrait d'au moins une bactérie filamenteuse non photosynthétique et un extrait de cellules d'au moins un végétal de la famille des Iridacées.

16. Composition selon la revendication 15, caractérisée en ce que l'extrait de cellules d'au moins un végétal de la famille des Iridacées est un extrait de cellules de plante entière cultivée *in vivo*.

17. Composition selon l'une quelconque des revendications 15 ou 16, caractérisée en ce que l'extrait de cellules d'au moins un végétal de la famille des Iridacées est un extrait de matériel végétal obtenu par culture *in vitro*.

18. Composition selon l'une quelconque des revendications 15 à 17, caractérisée en ce que les cellules sont des cellules indifférenciées.

19. Composition selon l'une quelconque des revendications 15 à 18, caractérisée en ce que ledit extrait d'Iridacées est un extrait de matériel végétal provenant d'Iridacées d'un genre choisi parmi Romulea, Crocus, Iris, Gladiolus, Sisyrinchium et Hermodactylus.

20. Composition selon l'une quelconque des revendications 15 à 19, caractérisée en ce que ledit extrait d'Iridacées est un extrait de matériel végétal provenant d'Iris.

21. Composition selon l'une quelconque des revendications 15 à 20, caractérisée en ce que ledit extrait d'Iridacées est un extrait de matériel végétal provenant d'*Iris pallida*.

22. Composition cosmétique selon l'une quelconque des revendications 15 à 21, caractérisée en ce que ledit extrait d'Iridacées est utilisé en une quantité représentant de 0,001% à 20% du poids total de la composition et préférentiellement en une quantité représentant de 0,01% à 10% du poids total de la composition.

23. Composition pharmaceutique selon l'une quelconque des revendications 15 à 21, caractérisée en ce que ledit extrait d'Iridacées est utilisé en une quantité représentant de 0,01% à 30% du poids total de la composition et préférentiellement en une quantité représentant de 0,5% à 20% du poids total de la composition.

24. Composition cosmétique ou pharmaceutique, caractérisée en ce qu'elle comprend, dans un milieu cosmétiquement ou pharmaceutiquement acceptable, un extrait d'au moins une bactérie filamenteuse non photosynthétique et un composé diminuant la synthèse, la libération et/ou l'activité d'au moins un médiateur de l'inflammation à l'exception des agents anti-inflammatoires stéroïdiens et non-stéroïdiens.

25. Composition selon la revendication 24, caractérisée en ce que le composé diminuant la synthèse, la libération et/ou l'activité d'au moins un médiateur de l'inflammation est un composé diminuant la synthèse, la libération et/ou l'activité d'au moins un médiateur de l'inflammation cutanée.

26. Composition selon l'une quelconque des revendications 24 ou 25, caractérisée en ce que le composé diminuant la synthèse, la libération et/ou l'activité d'au moins un médiateur de l'inflammation est choisi parmi les antagonistes de substance P et/ou de CGRP, les inhibiteurs de NO-synthase, les antagonistes de bradykinine, les antagonistes de cytokines, les antagonistes d'histamine, les antagonistes du facteur de nécrose tumorale de type $\alpha$ (TNF$\alpha$).

27. Composition selon la revendication 26, caractérisée en ce que les antagonistes sont des antagonistes réceptoriels.

28. Composition selon l'une quelconque des revendications 26 ou 27, caractérisée en ce que l'antagoniste de substance P est choisi parmi les substances assurant une diminution de l'extravasation du plasma au travers de la paroi vasculaire induite par la capsaïcine ou par une stimulation nerveuse antidromique et les substances provoquant une inhibition de la contraction des muscles lisses induites par l'administration de substance P.

29. Composition selon l'une quelconque des revendications 26 à 28, caractérisée en ce que l'antagoniste de substance P est choisi parmi les peptides, les composés comprenant au moins un hétérocycle, les composés azotés comprenant au moins un cycle benzénique, les sels de cations monovalents divalents et trivalents, les eaux thermales et leurs mélanges.

30. Composition selon la revendication 29, caractérisée en ce que le peptide est le sendide ou le spantide II.

31. Composition selon la revendication 29, caractérisée en ce que le composé comprenant au moins un hétérocycle est un composé hétérocyclique azoté oxygéné ou soufré choisi parmi les dérivés de 2-tricyclyl-2-amino-éthane, les dérivés de spirolactame, les dérivés de quinuclidine, les dérivés azacycliques, les dérivés d'aminopyrrolidine, les dérivés de pipéridine, les aminoazahétérocycles, les dérivés d'isoindole, les dérivés du furanne, les dérivés du benzofuranne, les dérivés du thiophène et les dérivés du benzothiophène, et notamment les tétrazolyl-benzofuranne-carboxamides ou les tétrazolyl-benzothiophène-carboxamides.

**32.** Composition selon la revendication 29, caractérisée en ce que le sel est choisi parmi les chlorures, acétates, carbonates, bicarbonates, salicylates, borates, nitrates, hydroxydes, sulfates, persulfates, des glycérophosphates, les sels d'α-hydroxy-acides, les sels d'acides de fruits, les sels d'acides aminés et les sels d'acides gras de strontium, de magnésium, de lanthanides de numéro atomique allant de 57 à 71, de cobalt, de nickel, de manganèse, de baryum, d'yttrium, de cuivre, d'étain, de rubidium, de lithium et de zinc.

**33.** Composition selon la revendication 32, caractérisée en ce que le sel est choisi parmi les sels de strontium.

**34.** Composition selon l'une quelconque des revendications 32 ou 33, caractérisée en ce que le sel est du chlorure ou du nitrate de strontium.

**35.** Composition selon la revendication 29, caractérisée en ce que l'eau thermale est une eau provenant d'une source du bassin de Vichy.

**36.** Composition selon la revendication 35, caractérisée en ce que l'eau thermale provient des sources Célestins, Chomel, Grande-Grille, Hôpital, Lucas et Parc.

**37.** Composition selon l'une quelconque des revendications 35 ou 36, caractérisée en ce que l'eau thermale provient de la source Lucas.

**38.** Composition selon l'une quelconque des revendications 26 ou 27, caractérisée en ce que l'antagoniste de CGRP est choisi parmi les substances assurant une diminution de la vasodilatation induite par la capsaïcine ou par une stimulation électrique antidromique (appliquée sur un nerf afférent) et/ou une inhibition de la libération de CGRP par les fibres nerveuses sensitives et les substances provoquant une inhibition de la contraction du muscle lisse du vas deferens induite par le CGRP.

**39.** Composition selon la revendication 38, caractérisée en ce que l'antagoniste de CGRP est choisi parmi le CGRP 8-37 (séquence des acides aminés 8 à 37 de la partie N-terminale du CGRP) ou les anticorps anti-CGRP.

**40.** Composition selon la revendication 26, caractérisée en ce que l'inhibiteur de NO-synthase est choisi parmi les substances qui permettent *in situ* sur l'homme d'inhiber partiellement, voire totalement, la synthèse de monoxyde d'azote (NO).

**41.** Composition selon la revendication 40, caractérisée en ce que l'inhibiteur de NO-synthase est choisi parmi les composés inhibant la synthèse et/ou accélérant le catabolisme de la NO-synthase, les composés neutralisant la NO-synthase ou les composés intervenant en diminuant le signal transduit par la NO-synthase.

**42.** Composition selon l'une quelconque des revendications 40 ou 41, caractérisée en ce que l'inhibiteur de la NO-synthase est choisi parmi des peptides, synthétiques ou naturels, éventuellement modifiés, des molécules chimiques, synthétiques ou naturelles, des acides nucléiques antisens, des ribozymes, des anticorps anti-NO-synthase.

**43.** Composition selon l'une quelconque des revendications 40 à 42, caractérisée en ce que l'inhibiteur de la NO-synthase est choisi parmi la $N^G$-monométhyl-L-arginine (L-NMMA), la $N^G$-nitro-L-arginine, l'ester méthylé de la $N^G$-nitro-L-arginine, le chlorure de diphénylèneiodonium, la 7-nitroindazole, la N(5)-(1-iminoéthyl)-L-ornithine, la $N^G,N^G$-diméthyl-L-arginine, la $N^G,N^G$-diméthyl-arginine, le 2-(4-carboxyphényl)-4,4,5,5-tetraméthylimidazoline-1-oxyl-3-oxyde, l'aminoguanidine, la canavanine, l'ebselen et l'hormone stimulatrice des mélanocytes de type α.

**44.** Composition selon l'une quelconque des revendications 40 à 43, caractérisée en ce que l'inhibiteur de la NO-synthase est la $N^G$-monométhyl-L-arginine et l'hormone stimulatrice des mélanocytes de type α.

**45.** Composition selon l'une quelconque des revendications 26 ou 27, caractérisée en ce que l'antagoniste de la bradykinine est choisi parmi les composés inhibant la synthèse et/ou accélérant le catabolisme de la bradykinine, les composés neutralisant la bradykinine, les composés bloquant les récepteurs de la bradykinine tels que ceux qui interférent avec les effets de la bradykinine par leur fixation au récepteur de celle-ci (B1 ou B2), les composés inhibant la synthèse des récepteurs de la bradykinine ou les composés intervenant en diminuant le signal transduit par la bradykinine.

**46.** Composition selon la revendication 45, caractérisée en ce que l'antagoniste de la bradykinine est choisi parmi des peptides, synthétiques ou naturels, éventuellement modifiés, des molécules chimiques, synthétiques ou naturelles, des acides nucléiques antisens, des ribozymes, des anticorps anti-bradykinines, des récepteurs solubles de la bradykinine, des anticorps anti-récepteurs de la bradykinine ou des antagonistes des récepteurs de la bradykinine.

**47.** Composition selon l'une quelconque des revendications 45 ou 46, caractérisée en ce que l'antagoniste de la bradykinine est choisi parmi les composés qui interférent avec les effets de la bradykinine par leur fixation au récepteur de celle-ci (B1 ou B2) et préférentiellement au récepteur B2.

**48.** Composition selon l'une quelconque des revendications 45 à 47, caractérisée en ce que l'antagoniste de la bradykinine est choisi parmi la D-Arg, [Hyp3, D-Phe7]-bradykinin (NPC567), la [Thi 5, 8, D-Phe7]-bradykinin, la D-Arg, [Hyp3, Thi5,8, D-Phe7]-bradykinin, la N-$\alpha$-adamantaneacetyl-D-Arg, [Hyp3, Thi5,8, D-Phe7]-bradykinin, la des-Arg9, [Leu8]-bradykinin, la P-guanidobenzoyl, [Hyp3,Thi5,D-Tic7,Oic8]-bradykinin (S 16118), la D-Arg, [Hyp3, Thi5, D-Tic7,Oic8]-bradykinine (HOE 140), la D-Arg, [Hyp3, D-Hype (trans-propyl) 7, Oic 8]-bradykinin (NPC 17731).

**49.** Composition selon l'une quelconque des revendications 45 à 48, caractérisée en ce que l'antagoniste de la bradykinine est la D-Arg, [Hyp3, Thi5, D-Tic7,Oic8]-bradykinine (HOE 140).

**50.** Composition selon l'une quelconque des revendications 26 ou 27, caractérisée en ce que l'antagoniste d'histamine, de cytokines ou de TNF-$\alpha$ est une substance choisie parmi les antagonistes réceptoriels d'histamine, de cytokines ou de TNF-$\alpha$, ou parmi les antagonistes de la libération et/ou de la synthèse d'histamine, de cytokines ou de TNF-$\alpha$

**51.** Composition selon la revendication 50, caractérisée en ce que la substance choisie parmi les antagonistes réceptoriels d'histamine, de cytokines ou de TNF-$\alpha$ est une substance ayant une affinité sélective pour les récepteurs spécifiques de ces composés ou assurant une inhibition de la contraction des muscles lisses induites par l'administration d'histamine ou assurant une inhibition de l'adhésion de macrophages induite par les cytokines sur les cellules endothéliales ou une inhibition de la libération d'anions superoxydes induite par les cytokines sur les neutrophiles ou une inhibition de l'adhésion de macrophages induite par TNF-$\alpha$ sur les cellules endothéliales ou une inhibition de la libération d'anions superoxydes induite par TNF-$\alpha$ sur les neutrophiles ou inhibition de l'activité mitogène du TNF-$\alpha$ sur les fibroblastes du derme.

**52.** Composition selon l'une quelconque des revendications 50 ou 51, caractérisée en ce que l'antagoniste réceptoriel d'histamine est choisi parmi les dérivés de la diéthylène diamine comme la Cinnarizine, la cyclizine les dérivés de l'aminopropane comme la dexchloro-phéniramine, la triprolidine ; les dérivés de phénothiazine comme la prométhazine, l'alimémazine : ainsi que les composés cités pages 116 à 118 du livre Joseph R. Prous, The Year's Drug News, Therapeutic Targets, édition 1994, Prous Science Publishers comme la cétirizine HCl, l'ébastine, la loratadine, la sétastine HCl.

**53.** Composition selon la revendication 50, caractérisée en ce que l'antagoniste de libération de l'histamine est choisi parmi des composés hétérocycliques oxygénés ou soufrés tels que les dérivés du furanne, les dérivés du benzofuranne, les dérivés du thiophène et les dérivés du benzothiophène, comportant éventuellement des substituants azotés, préférentiellement les alcoxy- et/ou aryloxy- tétrazol-yl-benzofuranne-carboxamides ou les alcoxy- et/ou aryloxy-tétrazol-yl-benzothiophène-carboxamides.

**54.** Composition selon l'une quelconque des revendications 50 à 53, caractérisée en ce que l'inhibiteur de libération de l'histamine est choisi parmi le 5-méthoxy-3-phénoxy-N-1H-tétrazol-5-yl-benzothiophène-2-carboxamide, le 5-méthoxy-3-(1-méthyléthoxy)-N-1H-tétrazol-5-yl-benzothiophène-2-carboxamide, le 6-méthoxy-3-(1 méthyléthoxy)-N-1H-tétrazol-5-yl-benzothiophène-2-carboxamide, le 5-méthoxy-3-(1-méthyléthyl)-N-1H-tétrazol-5-yl-benzothiophène-2-carboxamide, le 3-benzyloxy-5-méthoxy-N-1H-tétrazol-5-yl-benzothiophène-2-carboxamide le 5-méthoxy-3-phénoxy-N-1H-tétrazol-5-yl-benzothiophène-2-carboxamide.

**55.** Composition selon l'une quelconque des revendications 26 ou 27, caractérisée en ce que l'antagoniste de cytokines est choisi parmi les antagonistes de la libération de l'interleukine 1 comme l'auranofine ou le SKF-105809 ou les antagonistes de la synthèse d'interleukine 1 comme la lactoférrine.

56. Composition selon la revendication 50, caractérisée en ce que l'antagoniste réceptoriel de TNF-α et les inhibiteurs de la libération et/ou de la synthèse de TNF-α sont choisis parmi la lisophyline, l'A802715, la sulfasalazine.

57. Composition cosmétique selon l'une quelconque des revendications 24 à 56, caractérisée en ce que le composé diminuant la synthèse, la libération et/ou l'activité d'au moins un médiateur de l'inflammation est en une quantité représentant de 0,0001% à 5% du poids total de la composition et préférentiellement en une quantité représentant de 0,001% à 2% du poids total de la composition.

58. Composition pharmaceutique selon l'une quelconque des revendications 24 à 56, caractérisée en ce que le composé diminuant la synthèse, la libération et/ou l'activité d'au moins un médiateur de l'inflammation est en une quantité représentant de 0,0001% à 20% du poids total de la composition et préférentiellement en une quantité représentant de 0,01% à 5% du poids total de la composition.

59. Composition selon l'une quelconque des revendications 14 à 58, caractérisée en ce qu'elle est destinée à traiter les désordres du système nerveux central, les troubles respiratoires, les syndromes allergiques, l'inflammation, la douleur, les désordres gastro-intestinaux, les désordres cutanés, les fibroses, les troubles de la maturation du collagène, les troubles cardio-vasculaires, les troubles vasospastiques, les désordres immunologiques et/ou les troubles du tractus urinaires.

60. Composition selon l'une quelconque des revendications 14 à 59, caractérisée en ce qu'elle est destinée à prévenir et/ou lutter contre les irritations cutanées et/ou les dartres et/ou les érythèmes et/ou les sensations dysesthésiques et/ou les sensations d'échauffement et/ou les prurits de la peau et/ou des muqueuses.

61. Composition selon l'une quelconque des revendications 14 à 60, caractérisée en ce qu'elle comprend en outre un agent choisi parmi les agents antibactériens, antiparasitaires, antifongiques, antiviraux, anti-inflammatoires, antiprurigineux, anesthésiques, anti-radicaux libres, anti-séborrhéiques, antipelliculaires, antiacnéiques et/ou les agents diminuant la différenciation et/ou la prolifération et/ou la pigmentation cutanée.

62. Composition selon l'une quelconque des revendications 14 à 61, caractérisée en ce que ladite bactérie appartient à l'ordre des Beggiatoales.

63. Composition selon l'une quelconque des revendications 14 à 62, caractérisée en ce que ladite bactérie appartient au genre Beggiatoa, Vitreoscilla, Flexithrix ou Leucothrix.

64. Composition selon l'une quelconque des revendications 14 à 63, caractérisée en ce que ladite bactérie est choisie parmi des souches de *Vitreoscilla filiformis*.

65. Composition cosmétique selon l'une quelconque des revendications 14 à 22 ou 25 à 57 ou 59 à 64, caractérisée en ce que ladite bactérie est en une quantité représentant de 0,0001% à 5% du poids total de la composition et préférentiellement en une quantité représentant de 0,001% à 1% du poids total de la composition.

66. Composition pharmaceutique selon l'une quelconque des revendications 14 à 21 ou 23 à 57 ou 58 à 64, caractérisée en ce que ladite bactérie est en une quantité représentant de 0,0001% à 10% du poids total de la composition et préférentiellement en une quantité représentant de 0,01% à 5% du poids total de la composition.

67. Procédé de traitement cosmétique, caractérisé en ce que l'on applique sur la peau, sur les cheveux et/ou sur les muqueuses une composition cosmétique telle que définie dans l'une quelconque des revendications 14 à 22 ou 24 à 57 ou 59 à 64.

**Claims**

1. Use of at least one extract from at least one non-photosynthetic filamentous bacterium as substance P antagonist in a cosmetic composition or for the preparation of a pharmaceutical composition.

2. Use of at least one extract from at least one non-photosynthetic filamentous bacterium in a cosmetic composition or for the preparation of a pharmaceutical composition intended for the treatment of disorders associated with an excess in the synthesis and/or in the release of substance P.

3. Use according to either one of Claims 1 and 2, characterized in that the composition is intended for the treatment of disorders of the central nervous system, respiratory disorders, allergic syndromes, inflammation, pain, gastrointestinal disorders, cutaneous disorders, fibroses, disorders of maturation of collagen, cardiovascular disorders, vasospastic disorders, immunological disorders and/or disorders of the urinary tract.

4. Use according to any one of Claims 1 to 3, characterized in that the cosmetic or pharmaceutical composition is intended for the treatment of sensitive skins.

5. Use according to any one of Claims 1 to 4, characterized in that the cosmetic or pharmaceutical composition is intended to prevent and/or combat cutaneous irritations and/or sores and/or erythemas and/or dysaesthetic sensations and/or warming sensations and/or pruritus of the skin and/or the mucous membranes.

6. Use according to any one of Claims 1 to 5, characterized in that the said bacterium belongs to the order of the Beggiatoales.

7. Use according to Claim 6, characterized in that the said bacterium belongs to the genus Beggiatoa, Vitreoscilla, Flexithrix or Leucothrix.

8. Use according to Claim 7, characterized in that the said bacterium is chosen from strains of *Vitreoscilla filiformis*.

9. Use in a cosmetic composition according to any one of Claims 1 to 8, characterized in that the said bacterial extract is used in an amount representing from 0.0001 % to 20 % of the total weight of the composition and preferably in an amount representing from 0.001 % to 10 % of the total weight of the composition.

10. Use in a pharmaceutical composition according to any one of Claims 1 to 8, characterized in that the said bacterial extract is used in an amount representing from 0.0001 % to 30 % of the total weight of the composition and preferably in an amount representing from 0.05 % to 20 % of the total weight of the composition.

11. Use according to one of Claims 1 to 10, characterized in that the cosmetic or pharmaceutical composition additionally comprises at least one product with an irritant effect.

12. Use according to one of Claims 1 to 11, characterized in that the cosmetic or pharmaceutical composition additionally comprises at least one extract from cells from at least one plant from the family of the Iridaceae.

13. Use according to one of Claims 1 to 12, characterized in that the cosmetic or pharmaceutical composition additionally comprises at least one compound which decreases the synthesis, the release and/or the activity of at least one mediator of inflammation.

14. Cosmetic or pharmaceutical composition, characterized in that it comprises, in a cosmetically or pharmaceutically acceptable medium, an extract from at least one non-photosynthetic filamentous bacterium and at least one product with an irritant effect chosen from <u>organic solvents</u> or active principles, such as α-hydroxy acids, β-hydroxy acids, α-keto acids, β-keto acids, retinoids, anthralins, anthranoids, peroxides, minoxidil, lithium salts, antimetabolites, keratolytics, vitamin D and its derivatives, hair dyes or colorants, perfuming alcoholic solutions, antiperspirant agents, depilatory active principles, permanent-wave active principles or depigmenting active principles.

15. Cosmetic or pharmaceutical composition, characterized in that it comprises, in a cosmetically or pharmaceutically acceptable medium, an extract from at least one non-photosynthetic filamentous bacterium and an extract from cells from at least one plant from the family of the Iridaceae.

16. Composition according to Claim 15, characterized in that the extract from cells from at least one plant from the family of the Iridaceae is an extract from cells from the whole plant cultivated *in vivo*.

17. Composition according to either one of Claims 15 and 16, characterized in that the extract from cells from at least one plant from the family of the Iridaceae is an extract from plant material obtained by *in vitro* culturing.

18. Composition according to any one of Claims 15 to 17, characterized in that the cells are undifferentiated cells.

19. Composition according to any one of Claims 15 to 18, characterized in that the said Iridacea extract is an extract from plant material originating from Iridaceae from a genus chosen- from Romulea, Crocus, Iris, Gladiolus, Sisyrinchium and Hermodactylus.

20. Composition according to any one of Claims 15 to 19, characterized in that the said Iridacea extract is an extract from plant material originating from Iris.

21. Composition according to any one of Claims 15 to 20, characterized in that the said Iridacea extract is an extract from plant material originating from *Iris pallida*.

22. Cosmetic composition according to any one of Claims 15 to 21, characterized in that the said Iridacea extract is used in an amount representing from 0.001 % to 20 % of the total weight of the composition and preferentially in an amount representing from 0.01 % to 10 % of the total weight of the composition.

23. Pharmaceutical composition according to any one of Claims 15 to 21, characterized in that the said Iridacea extract is used in an amount representing from 0.01 % to 30 % of the total weight of the composition and preferentially in an amount representing from 0.5 % to 20 % of the total weight of the composition.

24. Cosmetic or pharmaceutical composition, characterized in that it comprises, in a cosmetically or pharmaceutically acceptable medium, an extract from at least one non-photosynthetic filamentous bacterium and a compound which decreases the synthesis, the release and/or the activity of at least one mediator of inflammation, with the exception of steroidal and non-steroidal anti-inflammatory agents.

25. Composition according to Claim 24, characterized in that the compound which decreases the synthesis, the release and/or the activity of at least one mediator of inflammation is a compound which decreases the synthesis, the release and/or the activity of at least one mediator of cutaneous inflammation.

26. Composition according to either one of Claims 24 and 25, characterized in that the compound which decreases the synthesis, the release and/or the activity of at least one mediator of inflammation is chosen from substance P and/or CGRP antagonists, NO-synthase inhibitors, bradykinin antagonists, cytokine antagonists, histamine antagonists or $\alpha$-type tumour necrosis factor (TNF-$\alpha$) antagonists.

27. Composition according to Claim 26, characterized in that the antagonists are receptor antagonists.

28. Composition according to either one of Claims 26 and 27, characterized in that the substance P antagonist is chosen from substances providing a decrease in the extravasation of the plasma through the vascular wall induced by capsaicin or by an antidromic nerve stimulation and substances causing inhibition of the contraction of the smooth muscles induced by the administration of substance P.

29. Composition according to any one of Claims 26 to 28, characterized in that the substance P antagonist is chosen from peptides, compounds comprising at least one heterocycle, nitrogenous compounds comprising at least one benzene ring, salts of monovalent, divalent and trivalent cations, spring waters and their mixtures.

30. Composition according to Claim 29, characterized in that the peptide is sendide or spantide II.

31. Composition according to Claim 29, characterized in that the compound comprising at least one heterocycle is a nitrogen-containing, oxygen-containing or sulphur-containing heterocyclic compound chosen from 2-tricyclyl-2-aminoethane derivatives, spirolactam derivatives, quinuclidine derivatives, azacyclic derivatives, aminopyrrolidine derivatives, piperidine derivatives, aminoazaheterocycles, isoindole derivatives, furan derivatives, benzofuran derivatives, thiophene derivatives and benzothiophene derivatives and in particular tetrazolylbenzofurancarboxamides or tetrazolylbenzothiophenecarboxamides.

32. Composition according to Claim 29, characterized in that the salt is chosen from the chlorides, acetates, carbonates, bicarbonates, salicylates, borates, nitrates, hydroxides, sulphates, persulphates, glycerophosphates, $\alpha$-hydroxy acid salts, fruit acid salts, amino acid salts and fatty acid salts of strontium, magnesium, lanthanides with an atomic number ranging from 57 to 71, cobalt, nickel, manganese, barium, yttrium, copper, tin, rubidium, lithium and zinc.

33. Composition according to Claim 32, characterized in that the salt is chosen from strontium salts.

**34.** Composition according to either one of Claims 32 and 33, characterized in that the salt is strontium chloride or strontium nitrate.

**35.** Composition according to Claim 29, characterized in that the spring water is a water originating from a spring from the Vichy basin.

**36.** Composition according to Claim 35, characterized in that the spring water originates from the springs at Célestins, Chomel, Grande-Grille, Hôpital, Lucas and Parc.

**37.** Composition according to either one of Claims 35 and 36, characterized in that the spring water originates from the spring at Lucas.

**38.** Composition according to either one of Claims 26 and 27, characterized in that the CGRP antagonist is chosen from substances providing a decrease in the vasodilation induced by capsaicin or by an antidromic electrical stimulation (applied to an afferent nerve) and/or inhibition of the release of CGRP by sensitive nerve fibres and substances causing inhibition of the contraction of the smooth muscle of the vas deferens induced by CGRP.

**39.** Composition according to Claim 38, characterized in that the CGRP antagonist is chosen from CGRP 8-37 (sequence of the amino acids 8 to 37 of the N-terminal part of CGRP) or anti-CGRP antibodies.

**40.** Composition according to Claim 26, characterized in that the NO-synthase inhibitor is chosen from substances which make it possible *in situ* in man to partially or indeed completely inhibit the synthesis of nitrogen monoxide (NO).

**41.** Composition according to Claim 40, characterized in that the NO-synthase inhibitor is chosen from compounds which inhibit the synthesis and/or which accelerate the catabolism of NO-synthase, compounds which neutralize NO-synthase or compounds which are involved in decreasing the signal transduced by NO-synthase.

**42.** Composition according to either one of Claims 40 and 41, characterized in that the NO-synthase inhibitor is chosen from optionally modified, synthetic or natural peptides, synthetic or natural chemical molecules, antisense nucleic acids, ribozymes or anti-NO-synthase antibodies.

**43.** Composition according to any one of Claims 40 to 42, characterized in that the NO-synthase inhibitor is chosen from $N^G$-monomethyl-L-arginine (L-NMMA), $N^G$-nitro-L-arginine, the methyl ester of $N^G$-nitro-L-arginine, diphenyleneiodonium chloride, 7-nitroindazole, N(5)-(1-iminoethyl)-L-ornithine, $N^G,N^G$-dimethyl-L-arginine, $N^G,N^G$-dimethylarginine, 2-(4-carboxyphenyl)-4,4,5,5-tetramethylimidazoline-1-oxy 3-oxide, aminoguanidine, canavanine, ebselen and $\alpha$-type melanocyte-stimulating hormone.

**44.** Composition according to any one of Claims 40 to 43, characterized in that the NO-synthase inhibitor is $N^G$-mono-methyl-L-arginine and $\alpha$-type melanocyte-stimulating hormone.

**45.** Composition according to either one of Claims 26 and 27, characterized in that the bradykinin antagonist is chosen from compounds which inhibit the synthesis and/or which accelerate the catabolism of bradykinin, compounds which neutralize bradykinin, compounds which block bradykinin receptors, such as those which interfere with the effects of bradykinin by the binding thereof to the bradykinin receptor (B1 or B2), compounds which inhibit the synthesis of bradykinin receptors or compounds which are involved in decreasing the signal transduced by bradykinin.

**46.** Composition according to Claim 45, characterized in that the bradykinin antagonist is chosen from optionally modified, synthetic or natural peptides, synthetic or natural chemical molecules, antisense nucleic acids, ribozymes, anti-bradykinin antibodies, soluble bradykinin receptors, anti-bradykinin receptor antibodies or bradykinin receptor antagonists.

**47.** Composition according to either one of Claims 45 and 46, characterized in that the bradykinin antagonist is chosen from compounds which interfere with the effects of bradykinin by the binding thereof to the bradykinin receptor (B1 or B2) and preferentially to the B2 receptor.

**48.** Composition according to any one of Claims 45 to 47, characterized in that the bradykinin antagonist is chosen from D-Arg,[Hyp$^3$,D-Phe$^7$]-bradykinin (NPC567), [Thi$^{5,8}$,D-Phe$^7$]-bradykinin, D-Arg,[Hyp$^3$,Thi$^{5,8}$, D-Phe$^7$]-bradyki-

nin, N-$\alpha$-adamantane-acetyl-D-Arg,[Hyp$^3$,Thi$^{5,8}$,D-Phe$^7$]-bradykinin, des-Arg$^9$,[Leu$^8$]-bradykinin, P-guanidoben-zoyl, [Hyp$^3$,Thi$^5$,D-Tic$^7$,Oic$^8$]-bradykinin (S 16118), DArg,[Hyp$^3$,Thi$^5$,D-Tic$^7$,Oic$^8$]-bradykinin (HOE 140) or D-Arg,[Hyp$^3$,D-Hype(trans-propyl)$^7$,Oic$^8$]-bradykinin (NPC 17731).

49. Composition according to any one of Claims 45 to 48, characterized in that the bradykinin antagonist is D-Arg,[Hyp$^3$,Thi$^5$,D-Tic$^7$,Oic$^8$]-bradykinin (HOE 140).

50. Composition according to either one of Claims 26 and 27, characterized in that the histamine, cytokine or TNF-$\alpha$ antagonist is a substance chosen from histamine, cytokine or TNF-$\alpha$ receptor antagonists or from antagonists of the release and/or of the synthesis of histamine, of cytokines or of TNF-$\alpha$.

51. Composition according to Claim 50, characterized in that the substance chosen from histamine, cytokines or TNF-$\alpha$ receptor antagonists is a substance having a selective affinity for specific receptors for these compounds or providing inhibition of the contraction of the smooth muscles induced by the administration of histamine or ensuring inhibition of the adhesion of macrophages induced by cytokines with respect to endothelial cells or inhibition of the release of superoxide anions induced by cytokines with respect to neutrophiles or inhibition of the adhesion of mac-rophages induced by TNF-$\alpha$ with respect to endothelial cells or inhibition of the release of superoxide anions induced by TNF-$\alpha$ with respect to neutrophiles or inhibition of the mitogenic activity of TNF-$\alpha$ with respect to the fibroblasts of the dermis.

52. Composition according to either one of Claims 50 and 51, characterized in that the histamine receptor antagonist is chosen from diethylenediamine derivatives, such as cinnarizine or cyclizine; aminopropane derivatives, such as dexchlorpheniramine or triprolidine; phenothiazine derivatives, such as promethazine or alimemazine; and the compounds mentioned on pages 116 to 118 of the book by Joseph R. Prous, The Year's Drug News, Therapeutic Targets, 1994 edition, Prous Science Publishers, such as cetirizine HCl, ebastine, loratadine or setastine HCl.

53. Composition according to Claim 50, characterized in that the antagonist of the release of histamine is chosen from oxygen-containing or sulphur-containing heterocyclic compounds, such as furan derivatives, benzofuran derivatives, thiophene derivatives and benzothiophene derivatives, optionally containing nitrogenous substituents, and preferentially alkoxy- and/or aryloxytetrazolylbenzofurancarboxamides or alkoxy- and/or aryloxytetrazolylbenzothiophenecarboxamides.

54. Composition according to any one of Claims 50 to 53, characterized in that the inhibitor of the release of histamine is chosen from 5-methoxy-3-phenoxy-N-(1H-tetrazol-5-yl)benzothiophene-2-carboxamide, 5-methoxy-3-(1-methylethoxy)-N-(1H-tetrazol-5-yl)benzothiophene-2-carboxamide, 6-methoxy-3-(1-methylethoxy)-N-(1H-tetrazol-5-yl)benzothiophene-2-carboxamide, 5-methoxy-3-(1-methylethyl)-N-(1H-tetrazol-5-yl)benzothiophene-2-carboxamide, 3-benzyloxy-5-methoxy-N-(1H-tetrazol-5-yl)benzothiophene-2-carboxamide and 5-methoxy-3-phenoxy-N-(1H-tetrazol-5-yl)benzothiophene-2-carboxamide.

55. Composition according to either one of Claims 26 and 27, characterized in that the cytokine antagonist is chosen from antagonists of the release of interleukin-1, such as auranofin or SKF-105809, or antagonists of the synthesis of interleukin-1, such as lactoferrin.

56. Composition according to Claim 50, characterized in that the TNF-$\alpha$ receptor antagonist and the inhibitors of the release and/or of the synthesis of TNP-$\alpha$ are chosen from lisophyline, A802715 or sulphasalazine.

57. Cosmetic composition according to any one of Claims 24 to 56, characterized in that the compound which decreases the synthesis, the release and/or the activity of at least one mediator of inflammation is in an amount representing from 0.0001 % to 5 % of the total weight of the composition and preferentially in an amount representing from 0.001 % to 2 % of the total weight of the composition.

58. Pharmaceutical composition according to any one of Claims 24 to 56, characterized in that the compound which decreases the synthesis, the release and/or the activity of at least one mediator of inflammation is in an amount representing from 0.0001 % to 20 % of the total weight of the composition and preferentially in an amount representing from 0.01 % to 5 % of the total weight of the composition.

59. Composition according to any one of Claims 14 to 58, characterized in that it is intended for the treatment of disorders of the central nervous system, respiratory disorders, allergic syndromes, inflammation, pain, gastrointestinal disorders, cutaneous disorders, fibroses, disorders of maturation of collagen, cardiovascular disorders, vasospastic disorders, immunological disorders and/or disorders of the urinary tract.

60. Composition according to any one of Claims 14 to 59, characterized in that it is intended to prevent and/or combat cutaneous irritations and/or sores and/or erythemas and/or dysaesthetic sensations and/or warming sensations and/or pruritus of the skin and/or mucous membranes.

61. Composition according to any one of Claims 14 to 60, characterized in that it additionally comprises an agent chosen from antibacterial agents, agents for combating parasites, antifungal agents, antiviral agents, anti-inflammatory agents, antipruriginous agents, anaesthetic agents, agents for combating free radicals, antiseborrhoeic agents, antidandruff agents, antiacne agents and/or agents which decrease cutaneous pigmentation and/or proliferation and/or differentiation.

62. Composition according to any one of Claims 14 to 61, characterized in that the said bacterium belongs to the order of the Beggiatoales.

63. Composition according to any one of Claims 14 to 62, characterized in that the said bacterium belongs to the genus Beggiatoa, Vitreoscilla, Flexithrix or Leucothrix.

64. Composition according to any one of Claims 14 to 63, characterized in that the said bacterium is chosen from strains of *Vitreoscilla filiformis*.

65. Cosmetic composition according to any one of Claims 14 to 22 or 25 to 57 or 59 to 64, characterized in that the said bacterium is in an amount representing from 0.0001 % to 5 % of the total weight of the composition and preferentially in an amount representing from 0.001 % to 1 % of the total weight of the composition.

66. Pharmaceutical composition according to any one of Claims 14 to 21 or 23 to 57 or 58 to 64, characterized in that the said bacterium is in an amount representing from 0.0001 % to 10 % of the total weight of the composition and preferentially in an amount representing from 0.01 % to 5 % of the total weight of the composition.

67. Cosmetic treatment process, characterized in that a cosmetic composition as defined in any one of Claims 14 to 22 or 24 to 57 or 59 to 64 is applied on the skin, on the hair and/or on the mucous membranes.

**Patentansprüche**

1. Verwendung mindestens eines Extrakts mindestens einer fadenförmigen, nicht phototropen Bakterie als Antagonist der Substanz P in einer kosmetischen Zusammensetzung oder für die Herstellung einer pharmazeutischen Zusammensetzung.

2. Verwendung mindestens eines Extrakts mindestens einer fadenförmigen, nicht phototropen Bakterie in einer kosmetischen Zusammensetzung oder für die Herstellung einer pharmazeutischen Zusammensetzung, die für die Behandlung der Störungen bestimmt ist, die mit einer übermäßigen Synthese und/oder Freisetzung der Substanz P verbunden sind.

3. Verwendung nach einem der Ansprüche 1 und 2, dadurch gekennzeichnet, daß die Zusammensetzung für die Behandlung von Störungen des Zentralnervensystems, respiratorischen Beschwerden, allergischen Syndromen, Entzündungen, Schmerzen, Störungen im Magen-Darm-Trakt, Hautproblemen, Fibrosen, Störungen der Kollagenentwicklung, Herz-Kreislauf-Beschwerden, vasospastischen Störungen, immunologischen Störungen und/oder Beschwerden im Bereich der Harnröhre bestimmt ist.

4. Verwendung nach einem der Ansprüche 1 bis 3, dadurch gekennzeichnet, daß die kosmetische oder pharmazeutische Zusammensetzung für die Behandlung von empfindlicher Haut bestimmt ist.

5. Verwendung nach einem der Ansprüche 1 bis 4, dadurch gekennzeichnet, daß die kosmetische oder pharmazeutische Zusammensetzung für die Verhinderung und/oder die Bekämpfung von Hautreizungen und/oder Flechten und/oder Erythemen und/oder dysästhesischen Empfindungen und/oder Hitzegefühlen und/oder Juckreiz auf der Haut und/oder den Schleimhäuten bestimmt ist.

EP 0 761 204 B1

6. Verwendung nach einem der Ansprüche 1 bis 5, dadurch gekennzeichnet, daß die Bakterie zur Ordnung der Beggiatoales gehört.

7. Verwendung nach Anspruch 6, dadurch gekennzeichnet, daß die Bakterie zur Gattung Beggiatoa, Vitreoscilla, Flexithrix oder Leucothrix gehört.

8. Verwendung nach Anspruch 7, dadurch gekennzeichnet, daß die Bakterie unter den Stämmen von *Vitreoscilla filiformis* ausgewählt wird.

9. Verwendung nach einem der Ansprüche 1 bis 8 in einer kosmetischen Zusammensetzung, dadurch gekennzeichnet, daß der Bakterienextrakt in einem Mengenanteil von 0,0001 bis 20 % des Gesamtgewichts der Zusammensetzung und vorzugsweise in einem Mengenanteil von 0,001 bis 10 % des Gesamtgewichts der Zusammensetzung verwendet wird.

10. Verwendung nach einem der Ansprüche 1 bis 8 in einer pharmazeutischen Zusammensetzung, dadurch gekennzeichnet, daß der Bakterienextrakt in einem Mengenanteil von 0,0001 bis 30 % des Gesamtgewichts der Zusammensetzung und vorzugsweise in einem Mengenanteil von 0,05 bis 20 % des Gesamtgewichts der Zusammensetzung verwendet wird.

11. Verwendung nach einem der Ansprüche 1 bis 10, dadurch gekennzeichnet, daß die kosmetische oder pharmazeutische Zusammensetzung außerdem mindestens eine Substanz mit reizender Wirkung enthält.

12. Verwendung nach einem der Ansprüche 1 bis 11, dadurch gekennzeichnet, daß die kosmetische oder pharmazeutische Zusammensetzung außerdem mindestens einen Extrakt von Zellen mindestens einer Pflanze aus der Familie der Iridaceae enthält.

13. Verwendung nach einem der Ansprüche 1 bis 12, dadurch gekennzeichnet, daß die kosmetische oder pharmazeutische Zusammensetzung außerdem mindestens eine Verbindung enthält, die die Synthese, Freisetzung und/oder Wirkung mindestens eines Entzündungsmediators verringert.

14. Kosmetische oder pharmazeutische Zusammensetzung, dadurch gekennzeichnet, daß sie in einem kosmetisch oder pharmazeutisch akzeptablen Medium einen Extrakt mindestens einer fadenförmigen, nicht phototropen Bakterie und mindestens eine Substanz mit reizender Wirkung enthält, das unter organischen Lösungsmitteln und Wirkstoffen wie α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten , Keratolytika, Vitamin D und dessen Derivaten, Haarfarben oder Haarfärbemitteln, parfümierenden alkoholischen Lösungen, Antitranspirantien, enthaarenden Wirkstoffen, Wirkstoffen für die Dauerwellverformung, depigmentierenden Wirkstoffen ausgewählt ist.

15. Kosmetische oder pharmazeutische Zusammensetzung, dadurch gekennzeichnet, daß sie in einem kosmetisch oder pharmazeutisch akzeptablen Medium einen Extrakt mindestens einer fadenförmigen, nicht phototropen Bakterie und einen Extrakt von Zellen mindestens einer Pflanze oder pflanzlichen Materials aus der Familie der Iridaceae enthält.

16. Zusammensetzung nach Anspruch 15, dadurch gekennzeichnet, daß der Extrakt von Zellen von mindestens einer Pflanze aus der Familie der Iridaceae ein Extrakt von Zellen ganzer, *in vivo* kultivierter Pflanzen ist.

17. Zusammensetzung nach einem der Ansprüche 15 und 16, dadurch gekennzeichnet, daß der Extrakt von Zellen von mindestens einer Pflanze aus der Familie der Iridaceae ein Extrakt von pflanzlichem Material ist, das durch *In-vitro*-Kultivierung erhalten wird.

18. Zusammensetzung nach einem der Ansprüche 15 bis 17, dadurch gekennzeichnet, daß die Zellen undifferenzierte Zellen sind.

19. Zusammensetzung nach einem der Ansprüche 15 bis 18, dadurch gekennzeichnet, daß der Iridaceae-Extrakt ein Extrakt von pflanzlichem Material ist, das von Iridaceae-Pflanzen einer Gattung stammt, die unter Romulea, Crocus, Iris, Gladiolus, Sisyrinchium und Hermodactylus ausgewählt ist.

20. Zusammensetzung nach einem der Ansprüche 15 bis 19, dadurch gekennzeichnet, daß der Iridaceae-Extrakt ein Extrakt von pflanzlichem Material ist, das aus der Gattung Iris stammt.

21. Zusammensetzung nach einem der Ansprüche 15 bis 20, dadurch gekennzeichnet, daß der Iridaceae-Extrakt ein Extrakt von pflanzlichem Material ist, das von *Iris pallida* stammt.

22. Kosmetische Zusammensetzung nach einem der Ansprüche 15 bis 21, dadurch gekennzeichnet, daß der Iridaceae-Extrakt in einem Mengenanteil von 0,001 bis 20 % des Gesamtgewichts der Zusammensetzung und vorzugsweise in einem Mengenanteil von 0,01 bis 10 % des Gesamtgewichts der Zusammensetzung verwendet wird.

23. Pharmazeutische Zusammensetzung nach einem der Ansprüche 15 bis 21, dadurch gekennzeichnet, daß der Iridaceae-Extrakt in einem Mengenanteil von 0,01 bis 30 % des Gesamtgewichts der Zusammensetzung und vorzugsweise in einem Mengenanteil von 0,5 bis 20 % des Gesamtgewichts der Zusammensetzung verwendet wird.

24. Kosmetische oder pharmazeutische Zusammensetzung, dadurch gekennzeichnet, daß sie in einem kosmetisch oder pharmazeutisch akzeptablen Medium einen Extrakt mindestens einer fadenförmigen, nicht phototropen Bakterie und eine Verbindung enthält, die die Synthese, die Freisetzung und/oder die Wirkung mindestens eines Entzündungsmediators verringert, wobei steroidische und nicht-steroidische entzündungshemmende Mittel ausgenommen sind.

25. Zusammensetzung nach Anspruch 24, dadurch gekennzeichnet, daß die Verbindung, die die Synthese, die Freisetzung und/oder die Wirkung mindestens eines Entzündungsmediators verringert, eine Verbindung ist, die die Synthese, die Freisetzung und/oder die Wirkung mindestens eines Mediators von Hautentzündungen ist.

26. Zusammensetzung nach einem der Ansprüche 24 und 25, dadurch gekennzeichnet, daß die Verbindung, die die Synthese, die Freisetzung und/oder die Wirkung mindestens eines Entzündungsmediators verringert, unter den Antagonisten der Substanz P und/oder der Substanz CGRP, den Inhibitoren der NO-Synthase, den Antagonisten von Bradykinin, den Antagonisten der Cytokine, den Antagonisten von Histamin, den Antagonisten des Tumor-Nekrose-Faktors vom $\alpha$-Typ (TNF-$\alpha$) ausgewählt ist.

27. Zusammensetzung nach Anspruch 26, dadurch gekennzeichnet, daß die Antagonisten Rezeptor-Antagonisten sind.

28. Zusammensetzung nach einem der Ansprüche 26 und 27, dadurch gekennzeichnet, daß der Antagonist der Substanz P ausgewählt ist unter den Substanzen, die eine Annahme der Extravasation von Plasma durch die Gefäßwand gewährleisten, die durch Capsaicin oder durch eine antidrome Nervenstimulierung hervorgerufen wird, und den Substanzen, die eine Hemmung der Kontraktion der glatten Muskulatur hervorrufen, die durch die Verabreichung der Substanz P verursacht wird.

29. Zusammensetzung nach einem der Ansprüche 26 bis 28, dadurch gekennzeichnet, daß der Antagonist der Substanz P unter Peptiden, Verbindungen mit mindestens einem Heterocyclus, stickstoffhaltigen Verbindungen mit mindestens einem Benzolring, Salzen einwertiger, zweiwertiger und dreiwertiger Kationen, Thermalwässern und deren Gemischen ausgewählt ist.

30. Zusammensetzung nach Anspruch 29, dadurch gekennzeichnet, daß es sich bei dem Peptid um Sendide oder Spantide II handelt.

31. Zusammensetzung nach Anspruch 29, dadurch gekennzeichnet, daß die Verbindung mit mindestens einem Heterocyclus eine stickstoffhaltige, sauerstoffhaltige oder schwefelhaltige heterocyclische Verbindung ist, die unter den Derivaten von 2-Tricyclyl-2-aminoethan, den Spirolactam-Derivaten, den Chinuclidin-Derivaten, den azacyclischen Derivaten, den Aminopyrrolidin-Derivaten, den Piperidin-Derivaten, den Aminoazaheterocyclen, den Isoindol-Derivaten, den Furan-Derivaten, den Benzofuran-Derivaten, den Thiophen-Derivaten und den Benzothiophen-Derivaten, insbesondere den Tetrazolylbenzofurancarboxamiden und den Tetrazolylbenzothiophencarboxamiden, ausgewählt ist.

32. Zusammensetzung nach Anspruch 29, dadurch gekennzeichnet, daß das Salz unter Chloriden, Acetaten, Carbonaten, Hydrogencarbonaten, Salicylaten, Boraten, Nitraten, Hydroxiden, Sulfaten, Persulfaten, Glycerophosphaten, den Salzen von $\alpha$-Hydroxysäuren, den Salzen von Fruchtsäuren, den Salzen von Aminosäuren und den Salzen von Fettsäuren mit Strontium, Magnesium, den Lanthaniden mit der Atomzahl von 57 bis 71, Cobalt, Nickel, Mangan, Barium, Yttrium, Kupfer, Zinn, Rubidium, Lithium und Zink ausgewählt ist.

33. Zusammensetzung nach Anspruch 32, dadurch gekennzeichnet, daß das Salz unter den Strontiumsalzen ausgewählt ist.

34. Zusammensetzung nach einem der Ansprüche 32 und 33, dadurch gekennzeichnet, daß das Salz Strontiumchlorid oder Strontiumnitrat ist.

35. Zusammensetzung nach Anspruch 29, dadurch gekennzeichnet, daß das Thermalwasser ein Wasser ist, das aus einer Quelle des Vichy-Beckens stammt.

36. Zusammensetzung nach Anspruch 35, dadurch gekennzeichnet, daß das Thermalwasser aus der Celestin-, Chomel-, Grand- Grille-, Hôpital-, Lucas- und/oder Parc-Quelle stammt.

37. Zusammensetzung nach einem der Ansprüche 35 und 36, dadurch gekennzeichnet, daß das Thermalwasser aus der Lucas-Quelle stammt.

38. Zusammensetzung nach einem der Ansprüche 26 und 27, dadurch gekennzeichnet, daß der Antagonist von CGRP ausgewählt ist unter den Substanzen, die eine Abnahme der Gefäßerweiterung gewährleisten, die durch Capsaicin oder durch eine antidrome elektrische Stimulierung (angewendet an einem afferenten Nerven) und/oder eine Hemmung der Freisetzung von CGRP durch die sensiblen Nervenfasern hervorgerufen wird, und den Substanzen, die eine Hemmung der durch CGRP hervorgerufenen Kontraktion der glatten Muskulatur des Vas deferens hervorrufen.

39. Zusammensetzung nach Anspruch 38, dadurch gekennzeichnet, daß der Antagonist von CGRP unter CGRP 8-37 (Sequenz der Aminosäuren 8 bis 37 des N-terminalen Teils von CGRP) und den Antikörpern gegen CGRP ausgewählt ist.

40. Zusammensetzung nach Anspruch 26, dadurch gekennzeichnet, daß der Inhibitor der NO-Synthase unter den Substanzen ausgewählt ist, mit denen es möglich ist, die Synthese von Stickstoffmonoxid (NO) *in situ* im Menschen teilweise oder sogar vollständig zu hemmen.

41. Zusammensetzung nach Anspruch 40, dadurch gekennzeichnet, daß der Inhibitor der NO-Synthase unter den Verbindungen, die die Synthese der NO-Synthase hemmen und/oder den Abbaustoffwechsel der NO-Synthase beschleunigen, den Verbindungen, die die NO-Synthase neutralisieren, und den Verbindungen, die dadurch einwirken, daß sie das durch die NO-Synthase umgewandelte Signal abschwächen, ausgewählt ist.

42. Zusammensetzung nach einem der Ansprüche 40 und 41, dadurch gekennzeichnet, daß der Inhibitor der NO-Synthase unter synthetischen und natürlichen, gegebenenfalls modifizierten Peptiden, synthetischen und natürlichen chemischen Molekülen, Antisense-Nukleinsäuren, Ribozymen, Antikörpern gegen NO-Synthase ausgewählt ist.

43. Zusammensetzung nach einem der Ansprüche 40 bis 42, dadurch gekennzeichnet, daß der Inhibitor der NO-Synthase unter $N^G$-Monomethyl-L-arginin (L-NMMA), $N^G$-Nitro-L-arginin, $N^G$-Nitro-L-argininmethylester, Diphenyleniodoniumchlorid, 7-Nitroindazol, N(5)-(1-Iminoethyl)-L-ornithin, $N^G,N^G$-Dimethyl-L-arginin, $N^G,N^G$-Dimethylarginin, 2-(4-Carboxyphenyl)-4,4,5,5-tetramethylimidazolin-1-oxyl-3-oxid, Aminoguanidin, Canavanin, Ebselen und dem die Melanocyten vom Typ $\alpha$ stimulierenden Hormon ausgewählt ist.

44. Zusammensetzung nach einem der Ansprüche 40 bis 43, dadurch gekennzeichnet, daß der Inhibitor der NO-Synthase $N^G$-Monomethyl-L-arginin und/oder das die Melanocyten vom Typ $\alpha$ stimulierende Hormon ist.

45. Zusammensetzung nach einem der Ansprüche 26 und 27, dadurch gekennzeichnet, daß der Antagonist von Bradykinin ausgewählt ist unter den Verbindungen, die die Synthese von Bradykinin hemmen und/oder den Abbaustoffwechsel von Bradykinin beschleunigen, den Verbindungen, die Bradykinin neutralisieren, den Verbindungen, die die Rezeptoren von Bradykinin blockieren, wie z.B. die Verbindungen, die die Wirkungen des Bradykinins stören, indem sie sich an den Rezeptor von Bradykinins binden (B1 oder B2), den Verbindungen, die die Synthese der Rezeptoren von Bradykinin hemmen, und den Verbindungen, die dadurch eingreifen, daß sie das durch Bradykinin umgewandelte Signal abschwächen ist.

**46.** Zusammensetzung nach Anspruch 45, dadurch gekennzeichnet, daß der Antagonist von Bradykinin unter synthetischen und natürlichen, gegebenenfalls modifizierten Peptiden, synthetischen und natürlichen chemischen Molekülen, Antisense-Nukleinsäuren, Ribozymen, Antikörpern gegen Bradykinin, löslichen Rezeptoren von Bradykinin, Antikörpern gegen die Rezeptoren von Bradykinin und Antagonisten der Rezeptoren von Bradykinin ausgewählt ist.

**47.** Zusammensetzung nach einem der Ansprüche 45 und 46, dadurch gekennzeichnet, daß der Antagonist von Bradykinin unter den Verbindungen ausgewählt ist, die die Wirkungen des Bradykinins stören, indem sie sich an den Rezeptor des Bradykinins (B1 oder B2) und vorzugsweise an den B2-Rezeptor binden.

**48.** Zusammensetzung nach einem der Ansprüche 45 bis 47, dadurch gekennzeichnet, daß der Antagonist von Bradykinin unter dem D-Arg,[Hyp3, D-Phe7]-bradykinin (NPC576), dem [Thi 5, 8, D-Phe7]-bradykinin, dem D-Arg,[Hyp3, Thi5,8, D-Pe7]-bradykinin, dem N-$\alpha$-Adamantanacetyl-D-Arg, [Hyp3, Thi5,8,D-Phe7]-bradykinin, dem des-Arg9,[Leu8]-bradykinin, dem P-Guanidobenzoyl,[Hyp3,Thi5, D-Tic7, Oic8]-bradykinin (S 16118), dem D-Arg, [Hyp3,Thi5,D-Tic7, Oic8]-bradykinin (HOE 140), dem D-Arg,[Hyp3, D-Hype (trans-propyl) 7, Oic 8]-bradykinin (NPC 17731) ausgewählt ist.

**49.** Zusammensetzung nach einem der Ansprüche 45 bis 48, dadurch gekennzeichnet, daß der Antagonist von Bradykinin D-Arg, [Hyp3, Thi5, D-Tic7, Oic8]-bradykinin (HOE 140) ist.

**50.** Zusammensetzung nach einem der Ansprüche 26 und 27, dadurch gekennzeichnet, daß der Antagonist von Histamin, der Cytokine oder von TNF-$\alpha$ eine Substanz ist, die unter den Rezeptor-Antagonisten von Histamin, der Cytokine und von TNF-$\alpha$ und unter den Antagonisten der Freisetzung und/oder der Synthese von Histamin, der Cytokine und von TNF-$\alpha$ ausgewählt ist.

**51.** Zusammensetzung nach Anspruch 50, dadurch gekennzeichnet, daß die unter den Rezeptor-Antagonisten von Histamin, der Cytokine und von TNF-$\alpha$ ausgewählte Substanz eine Substanz ist, die eine selektive Affinität für die für diese Verbindungen spezifischen Rezeptoren aufweist oder die eine Hemmung der durch die Verabreichung von Histamin hervorgerufenen Kontraktion der glatten Muskulatur gewährleistet oder eine Hemmung des durch die Cytokine hervorgerufen Anhaftens von Makrophagen an den Endothelialzellen oder eine Hemmung der durch die Cytokine bei den Neutrophilen hervorgerufenen Freisetzung von Superoxidanionen oder eine Hemmung des durch TNF-$\alpha$ hervorgerufenen Anhaftens von Makrophagen an den Endothelialzellen oder eine Hemmung der durch TNF-$\alpha$ bei den Neutrophilen hervorgerufenen Freisetzung von Superoxidanionen oder eine Hemmung der mitogenen Wirkung des TNF-$\alpha$ auf die Fibroblasten der Dermis gewährleistet.

**52.** Zusammensetzung nach einem der Ansprüche 50 und 51, dadurch gekennzeichnet, daß der Rezeptor-Antagonist von Histamin unter den Diethylendiamin-Derivaten wie Cinnarizin, Cyclizin, den Aminopropan-Derivaten wie Dexchloropheniramin, Triprolidin, den Phenothiazin-Derivaten wie Promethazin und Alimemazin sowie den auf den Seiten 116 bis 118 des Buches "Joseph R. Prous, The Year's Drug News, Therapeutic Targets, Auflage 1994, Prous Science Publishers" angegebenen Verbindungen wie Cetirizin HCl, Ebastin, Loratadin, Setastin HCl ausgewählt ist.

**53.** Zusammensetzung nach Anspruch 50, dadurch gekennzeichnet, daß der Antagonist der Freisetzung von Histamin unter heterocyclischen sauerstoffhaltigen oder schwefelhaltigen Verbindungen, wie z.B. Furan-Derivaten, Benzofuran-Derivaten, Thiophen-Derivaten und Benzothiophen-Derivaten, die gegebenenfalls stickstoffhaltige Substituenten tragen, vorzugsweise den Alkoxy- und/oder Aryloxytetrazolylbenzofurancarboxamiden und den Alkoxy- und/oder Aryloxytetrazolylbenzothiophencarboxamiden, ausgewählt ist.

**54.** Zusammensetzung nach einem der Ansprüche 50 bis 53, dadurch gekennzeichnet, daß der Inhibitor der Freisetzung von Histamin unter 5-Methoxy-3-phenoxy-N-1H-tetrazol-5-yl-benzothiophen-2-carboxamid, 5-Methoxy-3-(1-methylethoxy)-N-1H-tetrazol-5-yl-benzothiophen-2-carboxamid, 6-Methoxy-3-(1-methylethoxy)-N-1H-tetrazol-5-yl-benzothiophen-2-carboxamid, 5-Methoxy-3-(1-methylethyl)-N-1H-tetrazol-5-yl-benzothiophen-2-carboxamid, 3-Benzyloxy-5-methoxy-N-1H-tetrazol-5-yl-benzothiophen-2-carboxamid, 5-Methoxy-3-phenoxy-N-1H-tetrazol-5-yl-benzothiophen-2-carboxamid ausgewählt ist.

**55.** Zusammensetzung nach einem der Ansprüche 26 und 27, dadurch gekennzeichnet, daß der Antagonist der Cytokine unter den Antagonisten der Freisetzung von Interleukin 1 wie Auranofin und SKF-105809 und den Antagonisten der Synthese von Interleukin 1 wie Lactoferrin ausgewählt ist.

**56.** Zusammensetzung nach Anspruch 50, dadurch gekennzeichnet, daß der Rezeptor-Antagonist von TNF-$\alpha$ und die Inhibitoren der Freisetzung und/oder der Synthese von TNF-$\alpha$ unter Lisophylin, A802715, Sulfasalazin ausgewählt sind.

**57.** Kosmetische Zusammensetzung nach einem der Ansprüche 24 bis 56, dadurch gekennzeichnet, daß die Verbindung, die die Synthese, die Freisetzung und/oder die Wirkung mindestens eines Entzündungsmediators verringert, in einem Mengenanteil von 0,0001 bis 5 % des Gesamtgewichts der Zusammensetzung und vorzugsweise in einem Mengenanteil von 0,001 bis 2 % des Gesamtgewichts der Zusammensetzung enthalten ist.

**58.** Pharmazeutische Zusammensetzung nach einem der Ansprüche 24 bis 56, dadurch gekennzeichnet, daß die Verbindung, die die Synthese, die Freisetzung und/oder die Wirkung mindestens eines Entzündungsmediators verringert, in einem Mengenanteil von 0,0001 bis 20 % des Gesamtgewichts der Zusammensetzung und vorzugsweise in einem Mengenanteil von 0,01 bis 5 % des Gesamtgewichts der Zusammensetzung enthalten ist.

**59.** Zusammensetzung nach einem der Ansprüche 14 bis 58, dadurch gekennzeichnet, daß sie für die Behandlung von Störungen des Zentralnervensystems, respiratorischen Beschwerden, allergischen Syndromen, Entzündungen, Schmerzen, Störungen im Magen-Darm-Trakt, Hautproblemen, Fibrosen, Störungen der Kollagenentwicklung, Herz-Kreislauf-Beschwerden, vasospastischen Störungen, immunologischen Störungen und/oder Beschwerden im Bereich der Harnröhre vorgesehen ist.

**60.** Zusammensetzung nach einem der Ansprüche 14 bis 59, dadurch gekennzeichnet, daß sie für die Verhinderung oder Bekämpfung von Hautreizungen und/oder Flechten und/oder Erythemen und/oder und/oder dysästhesischen Empfindungen und/oder Hitzegefühlen und/oder Juckreiz auf der Haut und/oder den Schleimhäuten bestimmt ist.

**61.** Zusammensetzung nach einem der Ansprüche 14 bis 60, dadurch gekennzeichnet, daß sie außerdem ein Mittel enthält, das unter antibakteriellen Mitteln, antiparasitären Mitteln, fungiziden Mitteln, antiviralen Mitteln, entzündungshemmenden Mitteln, juckreizstillenden Mitteln, Anaesthetika, Mitteln gegen freie Radikale, Antiseborrhöika, Antischuppenmitteln, Aknemitteln und/oder Mitteln, die die Differenzierung und/oder die Proliferation und/oder die Pigmentierung der Haut veringern, ausgewählt ist.

**62.** Zusammensetzung nach einem der Ansprüche 14 bis 61, dadurch gekennzeichnet, daß die Bakterie zur Ordnung der Beggiatoales gehört.

**63.** Zusammensetzung nach einem der Ansprüche 14 bis 62, dadurch gekennzeichnet, daß die Bakterie zu der Gattung Beggiatoa, Vitreoscilla, Flexithrix oder Leucothrix gehört.

**64.** Zusammensetzung nach einem der Ansprüche 14 bis 63, dadurch gekennzeichnet, daß die Bakterie unter den Stämmen von *Vitreoscilla filiformis* ausgewählt ist.

**65.** Kosmetische Zusammensetzung nach einem der Ansprüche 14 bis 22 und 25 bis 57 und 59 bis 64, dadurch gekennzeichnet, daß die Bakterie in einem Mengenanteil von 0,0001 bis 5 % des Gesamtgewichts der Zusammensetzung und vorzugsweise in einem Mengenanteil von 0,001 bis 1 % des Gesamtgewichts der Zusammensetzung enthalten ist.

**66.** Pharmazeutische Zusammensetzung nach einem der Ansprüche 14 bis 21 und 23 bis 57 und 58 bis 64, dadurch gekennzeichnet, daß die Bakterie in einer Menge von 0,0001 bis 10 % des Gesamtgewichts der Zusammensetzung und vorzugsweise in einer Menge von 0,01 bis 5 % des Gesamtgewichts der Zusammensetzung enthalten ist.

**67.** Verfahren zur kosmetischen Behandlung, dadurch gekennzeichnet, daß eine kosmetische Zusammensetzung, die wie in einem der Ansprüche 14 bis 22 und 24 bis 57 und 59 bis 64 definiert ist, auf die Haut, die Haare und/oder die Schleimhäute aufgetragen wird.